# EUROPEAN PATENT APPLICATION

(11) **EP 4 763 842 A1**
(43) Date of publication of application: **24.06.2026**
(21) Application number: 25821241.4
(22) Date of filing: 10.06.2025
(51) Int. Cl.: C07D 411/14, H01M 10/0567

(54) **POLYCYCLIC COMPOUND, AND PREPARATION METHOD THEREFOR AND USE THEREOF, ELECTROLYTE, AND BATTERY**

(30) Priority: 13.06.2024 CN 202410757961
(71) Applicant: Guangzhou Tinci Materials Technology Co., Ltd., Guangzhou, Guangdong 510760 (CN); Zhejiang Tianshuo Fluorine Silicon New Materials Technology Co., Ltd., Quzhou, Zhejiang 324000 (CN)
(72) Inventor: XU, Wenjun, Guangzhou, Guangdong 510670 (CN); SUN, Anle, Guangzhou, Guangdong 510670 (CN); LIANG, Lina, Guangzhou, Guangdong 510670 (CN)
(74) Representative: Studio Torta S.p.A.
(86) International application number: PCT/CN2025/100268
(87) International publication number: WO 2025/256531

(57) **Abstract**

Provided are a polycyclic compound and a method for preparing the same, use, an electrolyte, and a battery. The polycyclic compound includes a compound represented by Formula I and a chlorine-containing organic compound. Based on a mass of the polycyclic compound, a content of the compound represented by the Formula I is greater than or equal to 99 wt%, and a total content of the chlorine-containing organic compound is less than or equal to 300 ppm. In the Formula I, R₁ and R₂ are each independently selected from any one of H, F, alkyl, or fluoroalkyl.

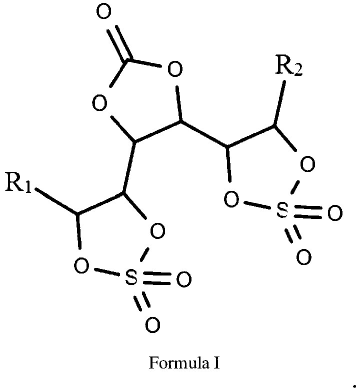

## Description

### CROSS-REFERENCE TO RELATED APPLICATION

This application claims priority to Chinese patent application No. 202410757961.0, titled "POLYCYCLIC COMPOUND AND METHOD FOR PREPARING THE SAME, USE, ELECTROLYTE, AND BATTERY", filed with China National Intellectual Property Administration on June 13, 2024, the entire contents of which are incorporated herein by reference.

### FIELD

The present disclosure belongs to the field of a battery, and more particularly, to a polycyclic compound and a method for preparing the same, use, an electrolyte, and a battery.

### BACKGROUND

With the rapid development of markets such as an electronic device, an electric vehicle, smart home, a power tool, and intelligent transportation, a demand for a battery is continuously increasing. Taking a lithium-ion battery as an example, the lithium-ion battery is widely used in consumer electronics, energy storage and power batteries, the smart home, and other fields due to advantages of high specific energy, long cycle life, and low self-discharge thereof. Under normal circumstances, a battery consists of a positive electrode plate, a negative electrode plate, a separator, and an electrolyte. The electrolyte usually includes a solvent, an electrolyte lithium salt, and an electrolyte additive. Performance of the battery can be improved by adding the electrolyte additive to the electrolyte. A content of free chloride ions in the electrolyte is strictly controlled, which is generally required to be less than 5 ppm. Free chloride ions not only easily induce corrosion of a positive electrode foil (such as an aluminum foil) but also easily lead to dissolution of transition metal ions from the positive electrode plate of the battery, affecting electrochemical performance of the battery. In the electrolyte, chloride ions mainly come from a lithium salt and various additives. Therefore, selecting an appropriate additive has an important impact on exertion of the electrochemical performance of the battery.

### SUMMARY

The present disclosure aims to solve one of the technical problems in the related art to some extent.

The technical solution of the present disclosure is completed by the inventor based on the following findings.

Free chloride ions in an electrolyte have a significant impact on battery performance. Currently, most technicians in the field focus on reducing a content of free chloride ions in the electrolyte. However, in practice, the performance still fails to reach an optimal level. It was found by the inventor of the present disclosure that a conventional method for detecting chloride ions in the electrolyte cannot detect a chlorine-containing organic compound with a certain specific structure. This is because these chlorine-containing organic compounds with the certain specific structure are difficult to lose chlorine under existing test conditions, which can make chlorine from these compounds exists in the form of chloride ions. Therefore, existing test methods cannot detect the chlorine-containing organic compound with the certain specific structure. As a result, control of a content of the chlorine-containing organic compound may be overlooked by those skilled in the art. Yet precisely these chlorine-containing compounds with the specific structures have a relatively significant negative impact on performance of the electrolyte. The reason may be as follows. A cell usually contains trace amounts of water and some residual alkaline substances. If an electrolyte additive contains excessive chlorine-containing organic impurities, after the electrolyte additive is used in the electrolyte, the chlorine-containing organic impurities are prone to hydrolysis under high-temperature and high-pressure conditions during battery operation-especially under the action of residual alkaline substances. This hydrolysis may lead to an increase in the content of free chloride in the electrolyte, which not only easily induces corrosion of a positive electrode foil (e.g., an aluminum foil) but also easily leads to dissolution of transition metal ions from a positive electrode plate of a battery. Therefore, not only structural stability of a positive electrode active material can be reduced, but also the dissolved transition metal ions can easily deposit on a surface of a negative electrode, resulting in continuous decomposition and regeneration of an SEI film and continuous consumption of active lithium. Both the dissolution of transition metal ions and the decomposition/regeneration of the SEI film may promote decomposition of the electrolyte, which in turn easily leads to battery capacity fading. In addition, issues such as increased polarization, caused by SEI film thickening, electrolyte consumption, or by-product blockage of lithium intercalation/deintercalation channels, and a higher short-circuit risk may occur. In this way, low-temperature discharge, high-temperature storage, and cycling performance are significantly affected. Even if a water content in the cell is controlled in accordance with relevant standards, the standards impose stricter requirements on the free chloride ions than on water. In other words, even if the water content in the cell is low, if the water content exceeds a level corresponding to a free chloride ion standard, the hydrolysis of chlorine-containing organic compound may easily cause the content of free chloride ions to exceed the standard, deteriorating performance.

In addition, as an important component of a lithium secondary battery, the electrolyte has a crucial impact on comprehensive performance of the battery. An additive is an important component of the electrolyte. A suitable additive can significantly improve electrochemical performance of the battery. It was also found by the inventor that applying a compound represented by Formula I as an additive to a lithium battery electrolyte can achieve a good improvement effect on the electrochemical performance of the battery. However, when preparing the compound represented by the Formula I using an existing synthesis process, problems such as a high impurity content in a target product, a low synthesis efficiency, and a low yield are common. For example, there is currently a method in which a hexahydric alcohol and a carbonate undergo transesterification under catalysis of a catalyst, followed by addition of sulfonyl chloride for recrystallization to obtain the target product. However, this method has poor selectivity, making it difficult to obtain a single target product. Also, the target product has a low yield and the high impurity content. Moreover, use of sulfonyl chloride as a reaction raw material easily leads to a high content of chlorine-containing organic impurities in the synthesized target product.

In view of the above, a first objective of the present disclosure is to provide a polycyclic sulfate product. Due to high purity, i.e., with a chlorine-containing organic compound content of less than 300 ppm, this product can reduce an impact of the chlorine-containing organic compound on the battery performance while improving the battery performance.

In a first aspect, the present disclosure provides a polycyclic compound. The polycyclic compound includes: a compound represented by Formula I; and a chlorine-containing organic compound. Based on a mass of the polycyclic compound, a content of the compound represented by the Formula I is greater than or equal to 99 wt%, and a total content of the chlorine-containing organic compound is less than or equal to 300 ppm. 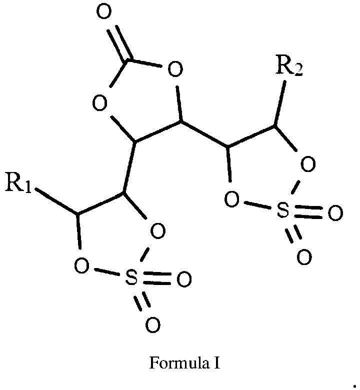 In the Formula I, R₁ and R₂ are each independently selected from any one of H, F, alkyl, or fluoroalkyl.

The above polycyclic compound of the present disclosure can provide the following advantageous effects. The compound represented by the Formula I may be used as the additive in the electrolyte, effectively improving the electrochemical performance of the battery. Specifically, not only formation of a dense CEI film on a positive electrode and a dense SEI film on a negative electrode can be facilitated, but also a small amount of inorganic salts can be formed in the CEI film generated by decomposition of the compound on the positive electrode (Taking the lithium battery as an example, these inorganic salts include Li₂SO₄ and/or Li₂O). As a result, rigidity and flexibility of a positive electrode interface layer can be enhanced, electrolyte decomposition can be inhibited, and stability of a positive electrode interface film can be improved. Moreover, the CEI film formed by the compound represented by the Formula I can also reduce an electrochemical impedance of the positive electrode, accelerate a transport rate of active ions (e.g., Li⁺), and help form a more stable electrode-electrolyte interface. In this way, a battery internal resistance and an interfacial reaction can be reduced, providing protection for the positive electrode. In addition, dissolution of transition metal ions from an active material of the positive electrode plate can be inhibited, reducing cyclic capacity fading of the battery, and improving cycling stability. Moreover, lithium carbonate is generated in the SEI film formed by the compound represented by the Formula I on the negative electrode. The lithium carbonate helps improve uniformity and stability of formation of the SEI film. In addition, the compound represented by the Formula I easily accepts electrons and is reduced, leading to cleavage of an S-O single bond. In a transition state formed after the cleavage, three oxygen atoms from O, S=O, and S=O on another cyclic sulfate group may form a claw-like spatial structure. This structure readily captures metal ions and forms a cage-like complex, which can stabilize the already dissolved transition metals, reduce a catalytic decomposition side reaction of transition metals on the SEI film, and inhibit a negative effect caused by deposition of transition metals in an elemental form after being reduced on the negative electrode. Furthermore, the compound represented by the Formula I has a low impedance, which is also beneficial for reducing a battery impedance. However, based on a conventional synthesis process for the compound represented by the Formula I, impurities are inevitably present, making it difficult to obtain a product with high purity and a low impurity content. In particular, an existing synthesis method using sulfonyl chloride as a reaction raw material generally may generate relatively high levels of chlorine-containing organic impurities. These chlorine-containing organic impurities are prone to hydrolysis, leading to an increase in the content of free chloride in the electrolyte. Therefore, not only corrosion of the positive electrode foil can occur, but also the dissolution of transition metal ions from the active material of the positive electrode plate can be induced, reducing improvement of the compound represented by the Formula I on the electrochemical performance of the battery. In the present disclosure, by using the polycyclic compound, with the content of the compound represented by the Formula I being greater than or equal to 99 wt% and the total content of chlorine-containing organic compound being less than or equal to 300 ppm, as the additive in the electrolyte, an increase in the content of free chloride in the electrolyte caused by introduction of the additive can be effectively reduced. Further, a risk of positive electrode foil corrosion and transition metal ion dissolution from the positive electrode plate, which is prone to occur due to an increased content of free chloride in the electrolyte, can be reduced. Therefore, a problem of a reduced improvement effect of the compound represented by the Formula I on the electrochemical performance of the battery, caused by presence of chlorine-containing organic impurities, can be effectively improved, enhancing the improvement effect of the polycyclic compound on the electrochemical performance of the battery.

In some embodiments, the chlorine-containing organic compound includes a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group.

In some embodiments, R₁ and R₂ are each independently selected from any one of H, F, or fluoromethyl.

In some embodiments, based on the mass of the polycyclic compound, the total content of the chlorine-containing organic compound is less than or equal to 100 ppm. Therefore, the problem of the reduced improvement effect of the compound represented by the Formula I on the electrochemical performance of the battery, caused by the presence of chlorine-containing organic impurities, can be effectively improved.

In some embodiments, based on the mass of the polycyclic compound, the content of the compound represented by the Formula I is greater than 99.9 wt%. Therefore, the improvement effect of the polycyclic compound on the electrochemical performance of the battery can be further enhanced.

In some embodiments, the chlorine-containing organic compound includes one or more of

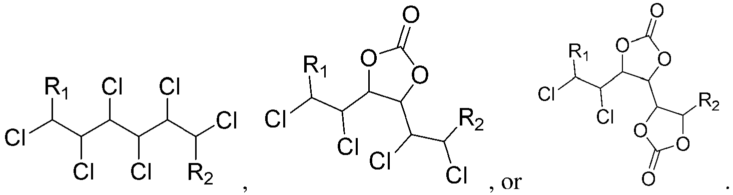

In some embodiments, the polycyclic compound further includes other organic compounds, the other organic compounds being selected from one or more of the following 6 compounds:

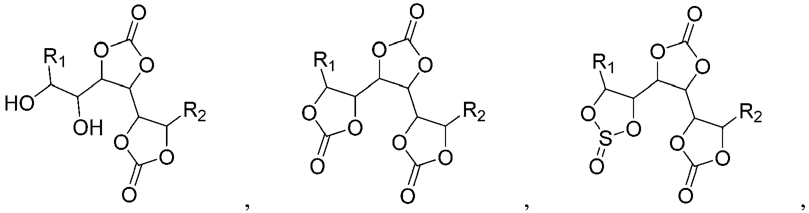

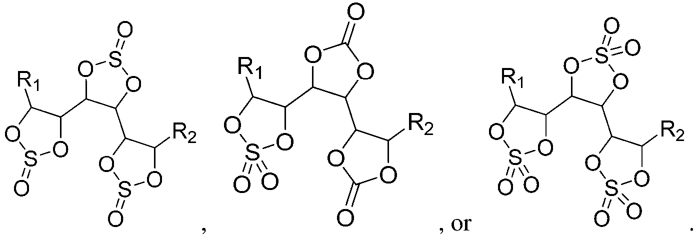

In some embodiments, the compound represented by the Formula I includes 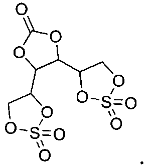 The compound has good stability, which is conducive to further enhancing the improvement effect of the polycyclic compound on the electrochemical performance of the battery.

In some embodiments, based on the mass of the polycyclic compound, a total content of the other organic compounds is less than or equal to 1000 ppm, optionally less than or equal to 200 ppm. Therefore, the problem of the reduced improvement effect of the compound represented by the Formula I on the electrochemical performance of the battery, caused by the impurities, can be further improved.

In some embodiments, based on the mass of the polycyclic compound, a total content of 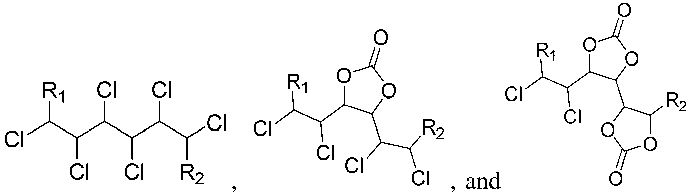 is less than or equal to 50 ppm. Therefore, the problem of the reduced improvement effect of the compound represented by the Formula I on the electrochemical performance of the battery, caused by the chlorine-containing organic impurities, can be further improved.

Based on the same inventive concept as the first aspect of the present disclosure, it is desired to obtain a polycyclic compound product with a chlorine-containing organic compound content being less than or equal to 300 ppm and purity of the compound represented by the Formula I being greater than or equal to 99 wt%. Based on this objective, a method for preparing a polycyclic compound according to a second aspect of the present disclosure is developed.

In a second aspect, the present disclosure provides a method for preparing the polycyclic compound. The method includes: (1) mixing a hexahydric alcohol, a monohydric alcohol, a carbonate, and a basic catalyst, and performing a transesterification reaction at a temperature ranging from 65°C to 100°C under a pressure controlled to be less than 2.5 MPa for 0.5 hours to 6 hours to obtain a compound 1; (2) dissolving the compound 1 in a solvent, and performing a condensation reaction with thionyl chloride to obtain a compound 2; and (3) reacting the compound 2 with an oxidizing agent to obtain the compound represented by the Formula I. Structural formulas of the hexahydric alcohol, the compound 1, and the compound 2 are represented by: 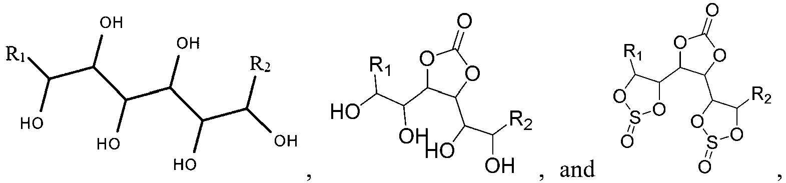 respectively. R₁ and R₂ are each independently selected from any one of H, F, Cl, alkyl, or fluoroalkyl.

In the present disclosure, the method for preparing the polycyclic compound can provide the following advantageous effects. In the transesterification reaction, reactivity of terminal hydroxyl groups (at the 1st position and at the 6th position) of the hexahydric alcohol can be inhibited by using a monohydric alcohol and under the pressure controlled to be less than 2.5 MPa. Subsequently, the reaction temperature is increased to range from 65°C to 100°C, which is a temperature higher than a boiling point of the monohydric alcohol (among the monohydric alcohols used in the present disclosure, methanol has a lowest boiling point at 65°C) by means of the pressure. Therefore, reactivity of middle hydroxyl groups (at the 2nd position, at the 3rd position, at the 4th position, and at the 5th position) of the hexahydric alcohol can be enhanced, resulting in the middle hydroxyl groups having significantly higher reactivity than the terminal hydroxyl groups. Also, under the conditions of heating and pressurization, a product formed by the reaction of the hydroxyl groups at the 3rd position and at the 4th position (i.e., the compound 1) has better symmetry and stability compared to a product formed by the reaction of hydroxyl groups at the 2nd position and at the 3rd position or a product formed by the reaction of hydroxyl groups at the 4th position and at the 5th position. Therefore, under these conditions, selectivity for the compound 1 and a conversion efficiency of the compound 1 in the transesterification reaction can be improved, yielding an intermediate product containing the compound 1 with a high conversion rate and high selectivity. In this way, on the one hand, a content of residual hexahydric alcohol in the transesterification reaction product can be effectively reduced. On the other hand, a content of impurities 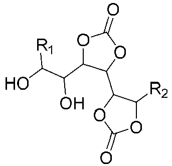 and 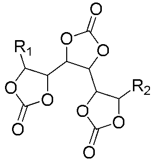 can be lowered. Further, a content of impurities 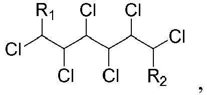 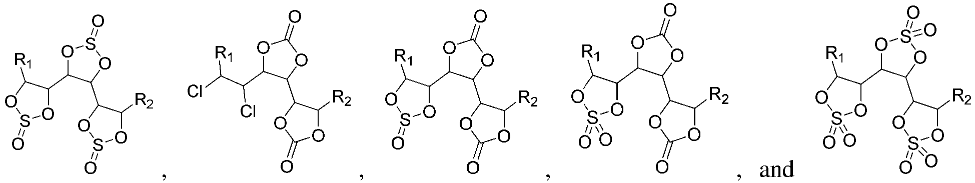 can be lowered, which may be generated when residual hexahydric alcohol or impurities participate in subsequent condensation reaction and oxidation reaction. In addition, no additional organic solvent is used in the transesterification reaction of the present disclosure. Carbonate and monohydric alcohol serve as both reactants and solvents, making the process not only environmentally friendly but also easy to operate. In summary, this method not only achieves high selectivity and a high conversion rate for the compound represented by the Formula I but also produces low levels of impurities. In this way, issues existing in the synthesis of the compound represented by the Formula I, such as a low reaction conversion rate, cumbersome reaction steps, high levels of impurity generation, and inconvenience for industrial production, can be effectively improved. Also, obtaining a target product, with the content of the chlorine-containing organic compound being less than or equal to 300 ppm and the purity of the compound represented by the Formula I being greater than or equal to 99 wt%, can be facilitated. Using the prepared target product represented by the Formula I in the electrolyte can effectively reduce the increase in free chloride content in the electrolyte. Further, the risk of positive electrode foil corrosion and transition metal ion dissolution from the positive electrode plate, which is prone to occur due to the increased content of free chloride in the electrolyte, can be further reduced. Therefore, the problem of the reduced improvement effect of the compound represented by the Formula I on the electrochemical performance of the battery, caused by presence of chlorine-containing organic impurities, can be effectively improved, thereby enhancing the improvement effect of the prepared polycyclic compound on the electrochemical performance of the battery.

In the present disclosure, since the monohydric alcohol is used as the reaction raw material, limited by the boiling point of the monohydric alcohol (among the monohydric alcohols used in the present disclosure, the methanol has the lowest boiling point at 65°C), the monohydric alcohol may evaporate and reflux when the temperature reaches the boiling point thereof under a normal pressure or a negative pressure in a reaction system of the present disclosure. In the reflux process, a temperature of the solution and a temperature of vapor may continuously adjust each other, and finally a dynamic equilibrium is achieved. It was found by the inventor that under this dynamic equilibrium, a temperature of the reaction system may stabilize around the boiling point of the monohydric alcohol, making it difficult to increase the temperature of the reaction system and thus limiting the reaction rate. However, the pressurization method in the present disclosure may not only cooperate with the monohydric alcohol to improve reaction selectivity, but also increase the boiling point of the monohydric alcohol. Therefore, the reaction temperature can also be appropriately increased, ultimately improving the reaction rate.

In some embodiments, the transesterification reaction is performed at a temperature ranging from 70°C to 90°C and a pressure ranging from 0.5 MPa to 2.2 MPa for 0.5 hours to 6 hours. Further, in some embodiments, the transesterification reaction is performed at a temperature ranging from 75°C to 85°C and a pressure ranging from 1 MPa to 2 MPa. Therefore, a relatively high raw material conversion rate and a suitable transesterification reaction rate can be balanced, thereby obtaining the compound 1 with high selectivity, a high yield, and a low impurity content.

In some embodiments, a molar ratio of the carbonate to the hexahydric alcohol ranges from 3:1 to 5:1. Therefore, not only obtaining the suitable transesterification reaction rate and the high conversion rate can be facilitated, but also an impurity content in the final product can be reduced.

In the present disclosure, the monohydric alcohol serves as the solvent on the one hand. An amount of the monohydric alcohol only needs to be sufficient to completely dissolve the reaction raw material such as mannitol and carbonate. Generally, when a molar ratio of the monohydric alcohol to the hexahydric alcohol is greater than or equal to 10, a dissolution requirement may be met. In the related art, it was mentioned that excessive addition of methanol as the solvent is acceptable for dissolving the reaction raw material. However, actually, it was found by the inventor that a content of the monohydric alcohol also affects selectivity of the transesterification reaction. If the content of the monohydric alcohol is too high (when the molar ratio of the monohydric alcohol to the hexahydric alcohol is greater than 20:1), the selectivity of the transesterification reaction may be reduced to a certain extent. Based on this, in some embodiments, the molar ratio of the monohydric alcohol to the hexahydric alcohol ranges from 10:1 to 20:1, preferably 12:1 to 18:1, and more preferably 14:1 to 16:1. Therefore, not only obtaining the suitable transesterification reaction rate and the high conversion rate can be facilitated, but also the impurity content in the final product can be reduced.

In some embodiments, based on a mass of the hexahydric alcohol, an amount of the basic catalyst ranges from 0.01 wt% to 5 wt%, optionally from 0.02 wt% to 0.5 wt%, and more preferably from 0.02 wt% to 0.1 wt%. Therefore, not only smooth progression of the transesterification reaction can be facilitated, but also a risk that an excessive amount of the basic catalyst leads to increased consumption of thionyl chloride and generation of a large amount of sulfur dioxide during the subsequent condensation reaction can be reduced.

In some embodiments, the hexahydric alcohol includes one or more of mannitol, sorbitol, or galactitol.

In some embodiments, the carbonate includes one or more of dimethyl carbonate, diethyl carbonate, ethyl methyl carbonate, or diphenyl carbonate.

In some embodiments, the monohydric alcohol includes one or more of methanol, ethanol, propanol, or butanol.

In some embodiments, the basic catalyst includes one or more of sodium hydroxide, potassium hydroxide, potassium carbonate, sodium carbonate, lithium carbonate, sodium methoxide, sodium ethoxide, sodium tert-butoxide, potassium tert-butoxide, triethylamine, or pyridine.

In some embodiments, subsequent to completion of the transesterification reaction, the method further includes: performing a reduced-pressure treatment to remove a low-boiling component. Therefore, purity of the compound represented by the Formula I can be further improved, reducing the impurity content.

In some embodiments, in the step (2), subsequent to completion of the condensation reaction, the method further includes: performing a reflux deacidification treatment. Therefore, the residual HCl generated from the condensation reaction can be reduced.

In some embodiments, in the step (2), a temperature of the reflux deacidification treatment rangs from 40°C to 80°C; and/or a duartion of the reflux deacidification treatment ranges from 10 minutes to 50 minutes. Meeting conditions of the reflux deacidification treatment is conducive to further reducing the residual HCl generated from the esterification reaction.

In some embodiments, in the step (2), subsequent to dissolving the compound 1 in the solvent, thionyl chloride is added dropwise to perform the condensation reaction. Therefore, formation of impurity 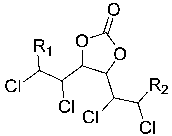 can be inhibited well.

In some embodiments, in the step (2), the condensation reaction is performed at a temperature ranging from 20°C to 100°C. Therefore, a reaction efficiency and purity of the final product can be balanced.

In some embodiments, in the step (2), a molar ratio of the compound 1 to the thionyl chloride is 1:(2 to 3). An addition duration of the thionyl chloride ranges from 0.5 hours to 2 hours. Therefore, not only the reaction rate can be balanced, but also purity of the compound 2 and the purity of the final product can be improved, reducing the impurity content.

In some embodiments, in the step (2), the solvent includes one or more of an ether solvent, N,N-dimethylformamide, N,N-dimethylacetamide, or an ester solvent.

In some embodiments, in the step (3), the oxidizing agent is provided as an aqueous solution. Subsequent to reacting the compound 2 with the oxidizing agent, the method further includes: performing a heating treatment on a reaction product to remove water.

In a third aspect, the present disclosure provides a polycyclic compound prepared by the above method for preparing the polycyclic compound. The prepared polycyclic compound includes a compound represented by Formula I and a chlorine-containing organic compound. A content of the compound represented by the Formula I is greater than or equal to 99 wt%. A total content of the chlorine-containing organic compound is less than or equal to 300 ppm. Characteristics and effects described for the above method for preparing the polycyclic compound are equally applicable to this polycyclic compound, and will not be repeated herein.

In a fourth aspect, the present disclosure provides use of the polycyclic compound according to the above or the above method for preparing the polycyclic compound in the field of electrolyte and battery.

In a fifth aspect, the present disclosure provides an electrolyte. The electrolyte includes an electrolyte additive. The electrolyte additive includes the polycyclic compound according to the above or the polycyclic compound prepared by the method according to the above. Using this electrolyte in the battery can well improve the electrochemical performance of the battery.

In some embodiments, based on a total mass of the electrolyte, a content of the electrolyte additive ranges from 0.1 wt% to 5 wt%.

In a sixth aspect, the present disclosure provides a battery. The battery includes the electrolyte according to the above.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and more understandable from the following description of embodiments taken in conjunction with the accompanying drawings.
FIG. 1 is a flow chart of preparation of a polycyclic compound according to an embodiment of the present disclosure.
FIG. 2 is a proton nuclear magnetic resonance spectrum of a final product prepared according to Example 1 of the present disclosure.
FIG. 3 is a carbon nuclear magnetic resonance spectrum of a final product prepared according to Example 1 of the present disclosure.
FIG. 4 is an LCMS mass spectrum of Sample 1, in which an upper panel shows a TIC graph, and a lower panel shows a mass spectrum bar graph at t=7.11 min.
FIG. 5 is an LCMS mass spectrum of Sample 1 and Cl₄, in which an upper panel shows a mass spectrum bar graph at t=7.11 min, and a lower panel shows a simulated mass spectrum bar graph of Cl₄.
FIG. 6 is a C-NMR spectrum of Sample 1.
FIG. 7 is an H-NMR spectrum of Sample 1.
FIG. 8 is an LCMS mass spectrum of Sample 2, in which an upper panel shows a TIC graph (black: a negative ion mode spectrum; red: a positive ion mode spectrum), and a lower panel shows a mass spectrum bar graph at t=4.83 min.
FIG. 9 is a C-NMR spectrum of Sample 2.
FIG. 10 is an H-NMR spectrum of Sample 2.
FIG. 11 is a GCMS TIC total ion chromatogram of Sample 3.
FIG. 12 is a GCMS library search result plot of Sample 3.
FIG. 13 is a liquid chromatogram of Sample A.
FIG. 14 is a liquid chromatogram of Sample B.

### DETAILED DESCRIPTION

Embodiments of the present disclosure are described below in detail, which are intended to explain, rather than limiting, the present disclosure.

In a first aspect, the present disclosure provides a polycyclic compound. The polycyclic compound includes: a compound represented by Formula I; and a chlorine-containing organic compound. Based on a mass of the polycyclic compound, a content of the compound represented by the Formula I is greater than or equal to 99 wt%, and a total content of the chlorine-containing organic compound is less than or equal to 300 ppm. 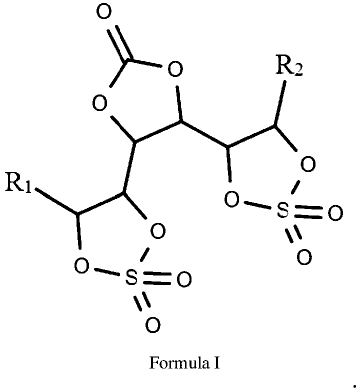 In the Formula I, R₁ and R₂ are each independently selected from any one of H, F, alkyl, or fluoroalkyl.

It should be noted that R₁ and R₂ may be the same or different. The alkyl and fluoroalkyl may each independently be an alkyl group or a fluoroalkyl group with 1 to 10 carbon atoms, and optionally the alkyl group or the fluoroalkyl group with 1 to 10 carbon atoms. In addition, the fluoroalkyl may be an alkyl group partially substituted with fluorine or fully substituted with fluorine. Exemplarily, based on the mass of the polycyclic compound, the content of the compound represented by the Formula I may be 99 wt%, 99.2 wt%, 99.4 wt%, 99.5 wt%, 99.7 wt%, 99.9 wt%, 99.92 wt%, 99.95 wt%, 99.97 wt%, or 99.99 wt%, and so on. The total content of the chlorine-containing organic compound may be less than or equal to 300 ppm, less than or equal to 280 ppm, less than or equal to 250 ppm, less than or equal to 220 ppm, less than or equal to 200 ppm, less than or equal to 180 ppm, less than or equal to 150 ppm, less than or equal to 120 ppm, less than or equal to 100 ppm, less than or equal to 80 ppm, less than or equal to 50 ppm, less than or equal to 40 ppm, less than or equal to 30 ppm, less than or equal to 20 ppm, less than or equal to 10 ppm, less than or equal to 5 ppm, and the like.

The compound represented by the Formula I may be used as an additive in an electrolyte to improve electrochemical performance of a battery. Specifically, in a battery formation stage, the compound represented by the Formula I tends to form a dense CEI film on a positive electrode and a dense SEI film on a negative electrode. The CEI film formed by decomposition of the compound on the positive electrode contains a small amount of inorganic salts (Taking a lithium battery as an example, these inorganic salts include Li₂SO₄ and/or Li₂O). As a result, rigidity and flexibility of a positive electrode interface layer can be enhanced, electrolyte decomposition can be inhibited, and stability of a positive electrode interface film can be improved. Moreover, the CEI film formed by the compound represented by the Formula I can also reduce an electrochemical impedance of the positive electrode, accelerate a transport rate of active ions (e.g., Li⁺), and help form a more stable electrode-electrolyte interface. In this way, a battery internal resistance and an interfacial reaction can be reduced, providing protection for the positive electrode. In addition, dissolution of transition metal ions from an active material of a positive electrode plate can be inhibited, reducing cyclic capacity fading of the battery, and improving cycling stability. Moreover, lithium carbonate is generated in the SEI film formed by the compound represented by the Formula I on the negative electrode. The lithium carbonate helps improve uniformity and stability of formation of the SEI film. In addition, the compound represented by the Formula I easily accepts electrons and is reduced, leading to cleavage of an S-O single bond. In a transition state formed after the cleavage, three oxygen atoms from O, S=O, and S=O on another cyclic sulfate group may form a claw-like spatial structure. This structure readily captures metal ions and forms a cage-like complex, which can stabilize the already dissolved transition metals, reduce a catalytic decomposition side reaction of transition metals on the SEI film, and inhibit a negative effect caused by deposition of transition metals in an elemental form after being reduced on the negative electrode. Furthermore, the compound represented by the Formula I has a low impedance, which is also beneficial for reducing a battery impedance.

Based on a conventional synthesis process for the compound represented by the Formula I, impurities are inevitably present, making it difficult to obtain a product with high purity and a low impurity content. In particular, an existing synthesis method using sulfonyl chloride as a reaction raw material generally may generate relatively high levels of chlorine-containing organic impurities. These impurity components include, but are not limited to a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group. These chlorine-containing organic impurities are prone to hydrolysis, leading to an increase in a content of free chloride in the electrolyte after the polycyclic compound is introduced into the electrolyte as the additive. Therefore, not only corrosion of the positive electrode foil can occur, but also the dissolution of transition metal ions from the active material of the positive electrode plate can be induced, reducing improvement of the compound represented by the Formula I on the electrochemical performance of the battery.

In the present disclosure, by using the polycyclic compound, with the content of the compound represented by the Formula I being greater than or equal to 99 wt% and the total content of chlorine-containing organic compound being less than or equal to 300 ppm, as the additive in the electrolyte, the increase in the content of free chloride in the electrolyte caused by introduction of the additive can be effectively reduced. Further, a risk of positive electrode foil corrosion and transition metal ion dissolution from the positive electrode plate, which is prone to occur due to an increased content of free chloride in the electrolyte, can be reduced. Therefore, a problem of a reduced improvement effect of the compound represented by the Formula I on the electrochemical performance of the battery, caused by presence of chlorine-containing organic impurities, can be effectively improved, enhancing the improvement effect of the polycyclic compound on the electrochemical performance of the battery.

In some specific embodiments of the present disclosure, when at least one of R₁ or R₂ is not H, synthesis of the compound represented by the Formula I may be performed by pre-obtaining an alcohol raw material with a corresponding substitution position and a substituent type, and then using these alcohol raw materials to synthesize the compound represented by the Formula I through processes such as a transesterification reaction and a condensation reaction.

In some specific embodiments of the present disclosure, R₁ and R₂ may be each independently selected from any one of H, F, or fluoromethyl. Therefore, not only the synthesis of the compound represented by the Formula I can be facilitated, but also introduction of a chlorine-containing compound in the additive can be further avoided, thereby reducing a potential increase in free chlorine content when the electrolyte additive is introduced into the electrolyte. When at least one of R₁ or R₂ is F, fluorine substitution or fluoromethyl substitution can be performed on terminal groups of the hexahydric alcohol, in which the terminal groups have higher reactivity and are more easily substituted. Then, the hexahydric alcohol with fluorine-substituted or fluoromethyl-substituted terminal groups is mixed with a raw material such as carbonate for the transesterification reaction, and a subsequent condensation reaction and a subsequent oxidation reaction.

In some specific embodiments of the present disclosure, R₁ and R₂ may be each independently H. That is, the compound represented by the Formula I may include 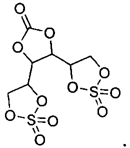 This compound has strong polarity and weak interaction with other non-polar molecules, resulting in less intermolecular friction and collision. Compared with other sulfate compounds, this compound has better stability. In addition, compared with other sulfate compounds, this compound also has better thermal stability. This compound may be stored at a room temperature without a need for a low-temperature. This compound may operate stably within a wide voltage range, and possess an excellent cycle life and capacity retention rate. Moreover, this compound has superior high-temperature performance, maintaining relatively stable electrochemical performance in a high-temperature environment, and thus prolonging a service life of the battery. Further, an oxidation potential and a reduction potential of this compound are both prior to those of a conventional electrolyte solvent. In the battery formation process, this compound is preferentially oxidized at the positive electrode and reduced at the negative electrode relative to the solvent, facilitating formation of the dense CEI film on the positive electrode and the dense SEI film on the negative electrode. Therefore, the improvement effect of the polycyclic compound on the electrochemical performance of the battery can be further enhanced.

In some specific embodiments of the present disclosure, based on the mass of the polycyclic compound, the total content of the chlorine-containing organic compound may be less than or equal to 100 ppm, for example, less than or equal to 100 ppm, less than or equal to 80 ppm, less than or equal to 50 ppm, less than or equal to 30 ppm, or in a range of 1 ppm to 99 ppm. Meeting the specified range can further reduce the increase in the free chlorine content in the electrolyte that may be caused by introduction of the polycyclic compound, further lowering the risk of positive electrode foil corrosion and transition metal ion dissolution from the positive electrode plate, which is prone to occur due to the increased content of free chloride in the electrolyte. Therefore, the problem of a reduced improvement effect of the compound represented by the Formula I on the electrochemical performance of the battery, caused by presence of the impurities, can be further improved, enhancing the improvement effect of the polycyclic compound on the electrochemical performance of the battery.

In some specific embodiments of the present disclosure, based on the mass of the polycyclic compound, the content of the compound represented by the Formula I may range from 99 wt% to 99.9 wt%, for example, 99 wt%, 99.1 wt%, 99.5 wt%, 99.8 wt%, 99.9 wt%, 99.95 wt%, or 99.99 wt%. Particularly when the content is greater than 99.9 wt%, the improvement effect of the polycyclic compound on the electrochemical performance of the battery can be further enhanced.

In some specific embodiments of the present disclosure, the chlorine-containing organic compound may include, but is not limited to a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group. In the synthesis process of the compound represented by the Formula I, the chlorine-containing organic compound may be generated due to excessive amounts of hexahydric alcohol, insufficient process conditions for the transesterification reaction and the condensation reaction, and the like. The above-mentioned several types of chlorine-containing organic compound impurities are commonly found in a target product when the compound represented by the Formula I is prepared using the hexahydric alcohol, the carbonate, and the thionyl chloride as the raw material. Reducing the content of the chlorine-containing organic compound is beneficial for further enhancing the improvement effect of the polycyclic compound on the electrochemical performance of the battery.

In some specific embodiments of the present disclosure, the chlorine-containing organic compound may include one or more of 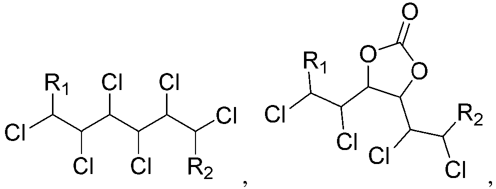 or 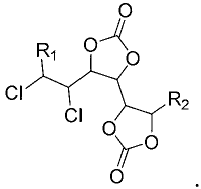 For example, when both R₁ and R₂ are H, the chlorine-containing organic compound may include one or more of 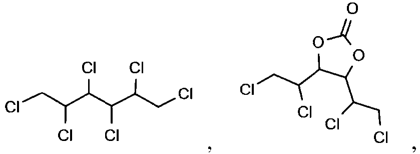 or 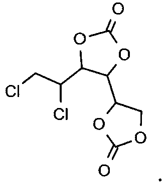 The three types of chlorine-containing organic compounds are common chlorine-containing organic impurities in the target product when the compound represented by the Formula I is prepared using the hexahydric alcohol, the carbonate, and the thionyl chloride as the raw material. In addition, the above-mentioned chlorine-containing organic compound is prone to hydrolysis to form free chlorine, which may increase the risk of positive electrode foil corrosion and transition metal ion dissolution from the positive electrode plate. Reducing a content of these three types of chlorine-containing organic compounds is conducive to further reducing the increase in the free chlorine content in the electrolyte that may be caused by the introduction of the polycyclic compound. In this way, the risk of transition metal ion dissolution from the positive electrode plate can be lowered, and the improvement effect of the polycyclic compound on the electrochemical performance of the battery can be enhanced. Further, based on the mass of the polycyclic compound, a total content of 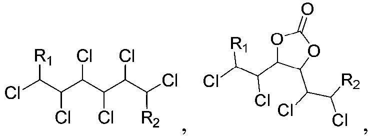 and 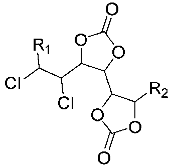 may be less than or equal to 50 ppm, for example, less than or equal to 45 ppm, less than or equal to 40 ppm, less than or equal to 35 ppm, less than or equal to 30 ppm, less than or equal to 20 ppm, less than or equal to 10 ppm, or less than or equal to 5 ppm. Therefore, the problem of the reduced improvement effect of the compound represented by Formula I on the electrochemical performance of the battery due to chlorine-containing organic impurities can be further improved.

In some specific embodiments of the present disclosure, the polycyclic compound further includes other organic compounds. The other organic compounds are selected from one or more of the following 6 compounds: 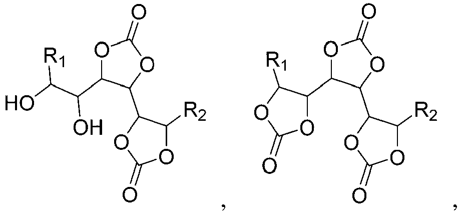 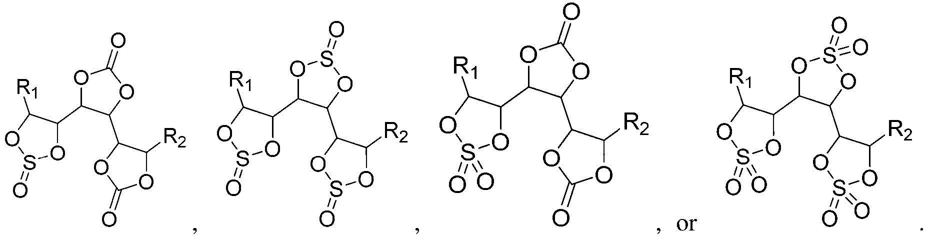 For example, when both R₁ and R₂ are H, the other organic compounds may be one or more of the following 6 compounds: 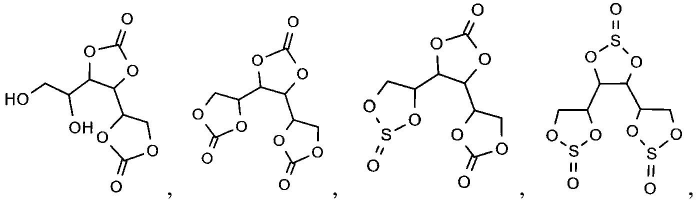 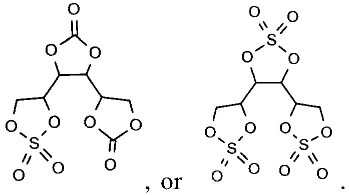 The several compounds mentioned above are also common impurities in the target product when the compound represented by the Formula I is prepared using the hexahydric alcohol, the carbonate, and the thionyl chloride as the raw material. Reducing the total content of the impurities is conducive to further enhancing the improvement effect of the polycyclic compound on the electrochemical performance of the battery. Based on the mass of the polycyclic compound, a total content of these 6 compounds may be less than or equal to 1000 ppm, which, for example, may be less than or equal to 900 ppm, less than or equal to 800 ppm, less than or equal to 700 ppm, less than or equal to 600 ppm, less than or equal to 500 ppm, less than or equal to 300 ppm, less than or equal to 200 ppm, less than or equal to 100 ppm, less than or equal to 50 ppm, or less than or equal to 30 ppm. Reducing the total content of these 6 impurity compounds helps to further reduce the risk of the reduced improvement effect of the compound represented by the Formula I on the electrochemical performance of the battery due to the presence of impurities. Controlling the total content of impurities to meet the above-mentioned range can further enhance the improvement effect of the polycyclic compound on the electrochemical performance of the battery. Further, based on the mass of the polycyclic compound, the total content of the 6 compounds may be less than or equal to 200 ppm.

Currently, in the common synthesis processes of the compound represented by the Formula I, there are problems such as poor selectivity of the target product, a low yield of the target product, and a large amount of impurities generated alongside the target product. In addition, there are also issues including an excessive solvent amount, a cumbersome process, and inconvenience for industrial production. Therefore, it is urgent to develop a method for synthesizing polycyclic sulfate that features a high conversion rate, a high yield, a relatively simple reaction step, and suitability for industrial production. Based on the same inventive concept as the first aspect of the present disclosure, it is desired to obtain a polycyclic compound product with a chlorine-containing organic compound content less than or equal to 300 ppm and purity of the compound represented by the Formula I greater than or equal to 99 wt%. The present disclosure provides a method for preparing a polycyclic compound with a high conversion rate, a high yield, and a low level of chlorine-containing organic impurities.

In a second aspect, the present disclosure provides a method for preparing a polycyclic compound. The method includes: mixing a hexahydric alcohol, a monohydric alcohol, a carbonate, and a basic catalyst, and performing a transesterification reaction at a temperature ranging from 65°C to 100°C under a pressure controlled to be less than 2.5 MPa for 0.5 hours to 6 hours to obtain a compound 1; (2) dissolving the compound 1 in a solvent, and performing a condensation reaction with thionyl chloride to obtain a compound 2; and (3) reacting the compound 2 with an oxidizing agent to obtain the compound represented by the Formula I. Structural formulas of the hexahydric alcohol, the compound 1, and the compound 2 are represented by: 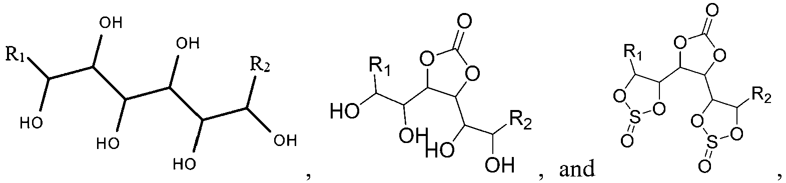 respectively. R₁ and R₂ are each independently selected from any one of H, F, Cl, alkyl, or fluoroalkyl.

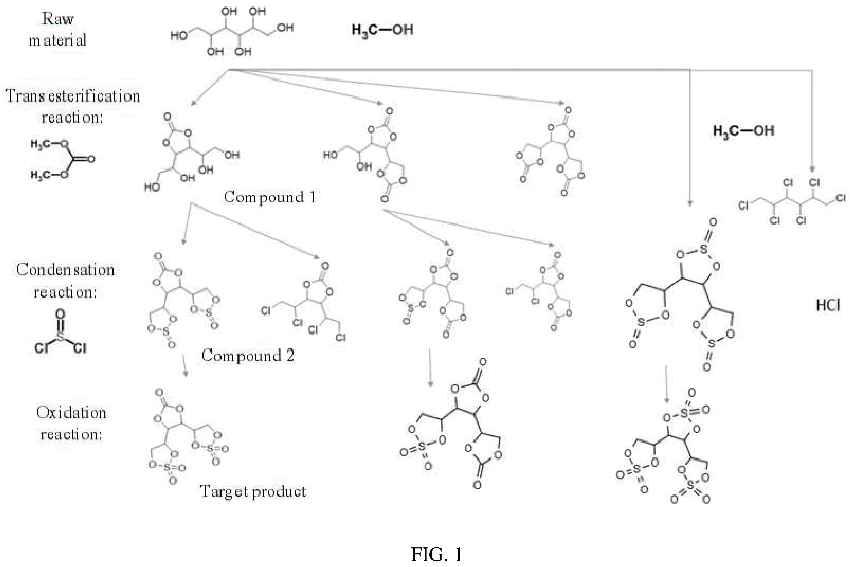

Exemplarily, FIG. 1 shows a flow chart of preparing the target product 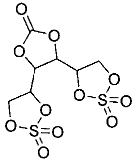 using 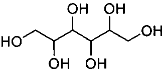 as the hexahydric alcohol, 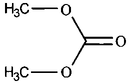 as the carbonate, H₃C-OH as the monohydric alcohol, and the thionyl chloride 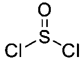 as the raw material. This figure also shows an intermediate product, the target product, and possible impurity components such as the chlorine-containing organic compound and other organic compounds in the preparation process.

As illustrated in FIG. 1, in the step (1), reactivity of terminal hydroxyl groups (at the 1st position and at the 6th position) of the hexahydric alcohol can be inhibited by using the monohydric alcohol and under the pressure controlled to be less than 2.5 MPa. Subsequently, the reaction temperature can be increased to range from 65°C to 100°C, which is a temperature higher than a boiling point of the monohydric alcohol (among the monohydric alcohols used in the present disclosure, methanol has a lowest boiling point at 65°C) by means of the pressure. Therefore, reactivity of middle hydroxyl groups (at the 2nd position, at the 3rd position, at the 4th position, and at the 5th position) of the hexahydric alcohol can be enhanced, resulting in the middle hydroxyl groups having significantly higher reactivity than the terminal hydroxyl groups. Also, under the conditions of heating and pressurization, a product formed by the reaction of the hydroxyl groups at the 3rd position and at the 4th position (i.e., the compound 1) have better symmetry and stability compared to a product formed by the reaction of hydroxyl groups at the 2nd position and at the 3rd position or a product formed by the reaction of hydroxyl groups at the 4th position and at the 5th position. Therefore, under these conditions, selectivity for the compound 1 and a conversion efficiency of the compound 1 in the transesterification reaction can be improved, yielding an intermediate product containing the compound 1 with a high conversion rate and high selectivity. Compared with a conventional process for synthesizing the target product represented by the Formula I, where selectivity is low and a by-product is easily generated, the process of the step (1) in the present disclosure can effectively reduce a content of residual hexahydric alcohol in the transesterification reaction product, and a content of impurities 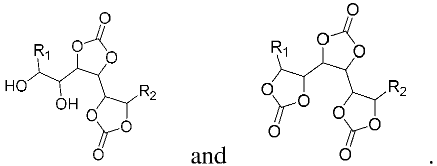 Further, reducing formation of impurities 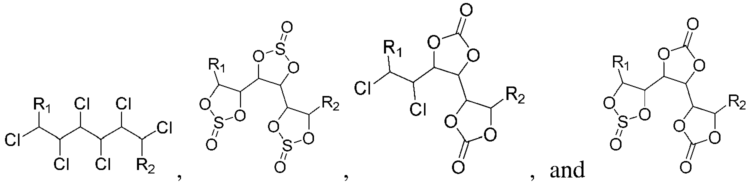 that may exist in a condensation reaction product of the step (2) can be facilitated, and a content of impurities 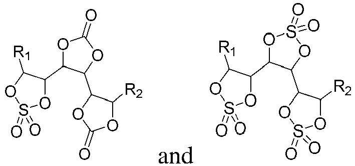 that may be generated in the subsequent oxidation reaction can be reduced.

**In** addition, in the step (1), no additional organic solvent is used in the transesterification reaction. Carbonate and monohydric alcohol serve as both reactants and solvents, making the process not only environmentally friendly but also easy to operate. The problems existing in the synthesis of the compound represented by the Formula I, such as a low reaction conversion rate, cumbersome reaction steps, high levels of impurity generation, and inconvenience for industrial production, can be effectively improved.

In summary, in the above embodiments of the present disclosure, the method for preparing the polycyclic compound not only achieves high selectivity and a high conversion rate for the compound represented by the Formula I but also produces low levels of impurities. In this way, issues existing in the synthesis of the compound represented by the Formula I, such as the low reaction conversion rate, cumbersome reaction steps, high levels of impurity generation, and inconvenience for industrial production, can be effectively improved. Also, obtaining the target product, with the content of chlorine-containing organic compound being less than or equal to 300 ppm and the purity of the compound represented by the Formula I being greater than or equal to 99 wt%, can be facilitated. Using the prepared target product represented by the Formula I in the electrolyte can effectively reduce the increase in free chloride content in the electrolyte. Further, the risk of positive electrode foil corrosion and transition metal ion dissolution from the positive electrode plate, which is prone to occur due to the increased content of free chloride in the electrolyte, can be further reduced. Therefore, the problem of the reduced improvement effect of the compound represented by the Formula I on the electrochemical performance of the battery, caused by presence of chlorine-containing organic impurities, can be effectively improved, enhancing an improvement effect of the prepared polycyclic compound on the electrochemical performance of the battery. It should be noted that the method for preparing the polycyclic compound in the second aspect of the present disclosure and the polycyclic compound in the first aspect of the present disclosure are provided based on the same inventive concept. Characteristics and effects described for the polycyclic compound in the first aspect of the present disclosure are also applicable to the method for preparing the polycyclic compound in the second aspect of the present disclosure, and thus details thereof will be omitted here.

As illustrated in FIG. 1, in the step (1), a temperature of the transesterification reaction may range from 65°C to 100°C, for example, 65°C, 70°C, 80°C, 90°C, or 100°C. A pressure of the transesterification reaction may be greater than an atmospheric pressure but less than 2.5 MPa, such as 0.2 MPa, 0.4 MPa, 0.6 MPa, 0.8 MPa, 1 MPa, 1.2 MPa, 1.4 MPa, 1.6 MPa, 1.8 MPa, 2 MPa, 2.2 MPa, or 2.5 MPa. A reaction duration of the transesterification reaction may range from 0.5 hours to 6 hours, for example, 0.5 h, 1 h, 2 h, 3 h, 4 h, 5 h, or 6 h. An excessively low pressure during the transesterification reaction (e.g., under a negative pressure) may cause a large amount of monohydric alcohol in the reaction system to be removed, which is unfavorable for improving selectivity of the transesterification reaction and inhibiting formation of the impurity 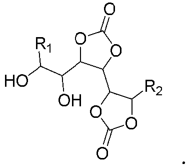 Conversely, an excessively high pressure during the transesterification reaction, as the reaction proceeds, for example, if a methanol content in the system is too high, which may easily hinder progress of the transesterification reaction, may lead to a reduced conversion rate and an increased residual raw material. In the present disclosure, controlling the temperature, the pressure, and the reaction duration of the transesterification reaction within the above range can balance a high raw material conversion rate and an appropriate transesterification reaction rate, obtaining the compound 1 with the high selectivity, the high yield, and the low impurity content.

Further preferably, in the step (1), the transesterification reaction may be performed at a temperature ranging from 70°C to 90°C and a pressure ranging from 0.5 MPa to 2.2 MPa. Further preferably, the transesterification reaction may be performed at a temperature ranging from 75°C to 850°C and a pressure ranging from 1 MPa to 2 MPa, which is conducive to further balancing the high raw material conversion rate and the appropriate transesterification reaction rate. Therefore, obtaining the compound 1 with higher selectivity, a higher yield, and a lower impurity content can be realized.

In some specific embodiments of the present disclosure, in the step (1), subsequent to completion of the transesterification reaction, the method further includes: performing a reduced-pressure treatment to remove a low-boiling component. In this way, purity of the compound represented by the Formula I in the final product can be improved, reducing the impurity content. For some specific examples, the reduced-pressure treatment may be a reduced-pressure distillation treatment. Optionally, a pressure for the reduced-pressure treatment may range from -90 kPa to -80 kPa (i.e., 90 kPa to 80 kPa lower than a standard atmospheric pressure), such as -90 kPa, -85 kPa, or -80 kPa. A temperature for the reduced-pressure treatment may range from 30°C to 60°C, such as 35°C, 40°C, 45°C, 50°C, or 55°C. Therefore, removal of the low-boiling component can be further facilitated.

In some specific embodiments of the present disclosure, as illustrated in FIG. 1, in the step (1), a molar ratio of the carbonate to the hexahydric alcohol may range from 3:1 to 5:1, such as 3/1, 3.5/1, 4/1, 4.5/1, or 5/1. Based on an amount of the hexahydric alcohol, appropriately increasing an amount of carbonate is beneficial to accelerating the progress of the transesterification reaction, improving a conversion rate of the raw material, and reducing a content of unreacted hexahydric alcohol in the intermediate product. Therefore, a content of impurities 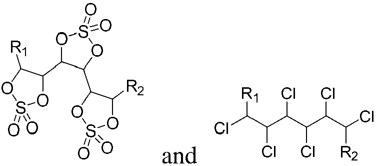 in the final product can be reduced. In the present disclosure, controlling the amount of carbonate within the above range enables the transesterification reaction to have an appropriate reaction rate. In this way, not only a utilization rate of the raw material and a yield of the compound 1 can be improved, and the content of impurities 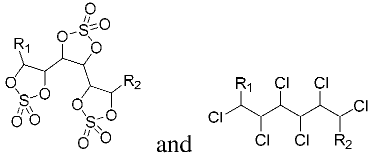 in the final product can be reduced; but also a risk of generation of impurities 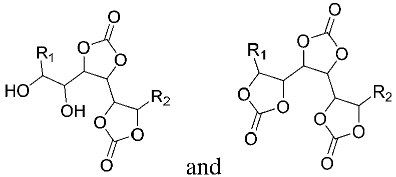 can be reduced. This risk is due to an excessive speed of the transesterification reaction caused by excessive carbonate addition, which leads to reaction of hydroxyl groups at terminal positions of hexahydric alcohol. Further, a content of impurities 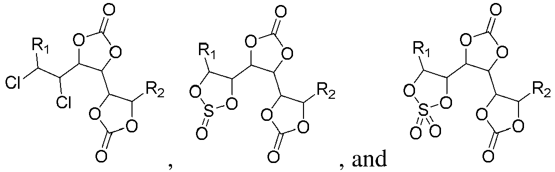 that may be generated subsequently can be reduced.

In some specific embodiments of the present disclosure, as illustrated in FIG. 1, in the step (1), a molar ratio of the monohydric alcohol to the hexahydric alcohol may range from 10:1 to 20:1, for example, 10/1, 12/1, 14/1, 16/1, 18/1, or 20/1. Further, preferably, the molar ratio of the monohydric alcohol to the hexahydric alcohol may range from 12:1 to 18:1. Even more preferably, the molar ratio of the monohydric alcohol to the hexahydric alcohol may range from 14:1 to 16:1. Controlling a relative amount of the monohydric alcohol to the hexahydric alcohol within the above range not only meets the requirement of using the monohydric alcohol as a solvent to dissolve the reaction raw material, but also reduces a risk that excessive monohydric alcohol may strongly inhibit the transesterification reaction. In this way, smooth progress of the transesterification reaction can be realized. In addition, the transesterification reaction can have the appropriate reaction rate. An issue where insufficient monohydric alcohol is unfavorable for improving the selectivity of the transesterification reaction can be mitigated, thereby preventing the terminal hydroxyl groups of the hexahydric alcohol from reacting to generate impurities 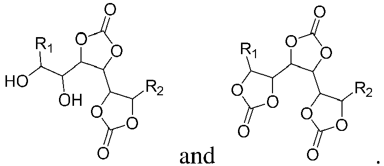 The contents of impurities 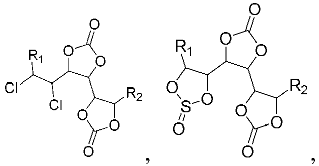 and 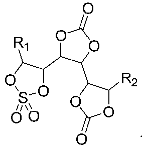 that may be generated subsequently can be further reduced.

In some specific embodiments of the present disclosure, in the step (1), based on a mass of the hexahydric alcohol, an amount of the basic catalyst ranges from 0.01 wt% to 5 wt%, for example, 0.01 wt%, 0.1 wt%, 0.5 wt%, 1 wt%, 2 wt%, 3 wt%, 4 wt%, or 5 wt%. Controlling the amount of the basic catalyst within the above range not only facilitates the smooth progress of the transesterification reaction, but also reduces a risk of excessive sulfur dioxide generation caused by an increased amount of the thionyl chloride in the subsequent condensation reaction due to an excessive basic catalyst.

In some specific embodiments of the present disclosure, in the step (1), based on a mass of the hexahydric alcohol, an amount of the basic catalyst ranges from 0.02 wt% to 0.5 wt%. Further preferably, based on the mass of the hexahydric alcohol, the amount of the basic catalyst may range from 0.02 wt% to 0.1 wt%. In the present disclosure, to facilitate industrialization, it is desirable to simplify a post-treatment process of each reaction step. Considering that the basic catalyst used in the transesterification reaction in the step (1) tends to make a post-reaction system alkaline, if no neutralization or purification process is performed, the basic catalyst may enter the condensation reaction process, consume thionyl chloride in the second step, and affect the condensation reaction. However, removing the basic catalyst may complicate a post-treatment process of the step (1). For example, an acidic substance such as oxalic acid may be required to be added to adjust a pH value, followed by post-treatment steps such as filtration and recrystallization. In the present disclosure, further reducing the amount of the basic catalyst can simplify the post-treatment step such as pH adjustment, filtration, and recrystallization after the step (1). However, a reduction in the catalyst content may easily affect a reaction rate and other parameters. In the present disclosure, through heating and pressurization, a drawback caused by a reduced catalyst amount can be mitigated, achieving a goal of simplifying the post-treatment process. On this basis, by further regulating relative amounts of the hexahydric alcohol, the monohydric alcohol, and the carbonate, and controlling the reaction temperature and the pressure within the above range, the drawback caused by the reduced catalyst amount can be further mitigated, achieving the goal of simplifying the post-treatment process.

In some specific embodiments of the present disclosure, in the step (1), when at least one of R₁ and R₂ in the hexahydric alcohol 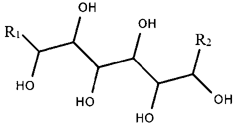 is not H, the target product represented by the Formula I may be synthesized by pre-obtaining a hexahydric alcohol raw material containing a corresponding substitution position and substituent type, and then using the hexahydric alcohol raw material to synthesize the compound represented by the Formula I through processes such as the transesterification reaction and the condensation reaction.

In some specific embodiments of the present disclosure, as illustrated in FIG. 1, in the step (1), the hexahydric alcohol may include, but is not limited to, one or more of mannitol, sorbitol, or galactitol. Selecting the hexahydric alcohol from the above group can help prepare a compound 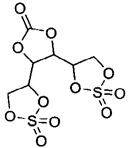 with better stability.

In some specific embodiments of the present disclosure, in the step (1), the carbonate may include, but is not limited to, one or more of dimethyl carbonate, diethyl carbonate, ethyl methyl carbonate, or diphenyl carbonate.

In some specific embodiments of the present disclosure, in the step (1), the monohydric alcohol may include, but is not limited to, one or more of methanol, ethanol, propanol, isopropanol, butanol, or tert-butanol.

In some specific embodiments of the present disclosure, in the step (1), the basic catalyst may include, but is not limited to, one or more of sodium hydroxide, potassium hydroxide, potassium carbonate, sodium carbonate, lithium carbonate, sodium methoxide, sodium ethoxide, sodium tert-butoxide, potassium tert-butoxide, triethylamine, or pyridine.

In some specific embodiments of the present disclosure, in the step (2), subsequent to completion of the condensation reaction, the method further includes: performing a reflux deacidification treatment. Therefore, residual HCl generated during the condensation reaction can be reduced, decreasing a potential content of free chlorine introduced into the electrolyte in subsequent processes. In some specific embodiments, a temperature of the reflux deacidification treatment may range from 40°C to 80°C, which, for example, may be 40 °C, 50 °C, 60 °C, 70 °C, or 80 °C. In some specific embodiments, a duartion of the reflux deacidification treatment may range from 10 minutes to 50 minutes, which, for example, may be 15 min, 20 min, 30 min, 40 min, or 50 min. Meeting conditions of the reflux deacidification treatment is beneficial for further reducing the residual HCl generated during the condensation reaction.

In some specific embodiments of the present disclosure, as illustrated in FIG. 1, in the step (2), subsequent to dissolving the compound 1 in the solvent, thionyl chloride is added dropwise to perform the condensation reaction. By slowly adding the thionyl chloride to the mixture to react with the compound 1 using this method, a side reaction potentially caused by an excess amount of thionyl chloride can be significantly reduced, effectively suppressing formation of the impurity

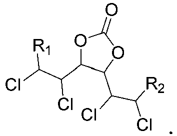

In some specific embodiments of the present disclosure, in the step (2), the condensation reaction may be performed at a temperature ranging from 20°C to 100°C, which, for example, may range from 20°C to 60 °C, or may be 20 °C, 25 °C, 30 °C, 50 °C, 60 °C, 80 °C, or 100 °C. Controlling the temperature of the condensation reaction to meet the above conditions not only helps reduce a risk of increased reactivity of the thionyl chloride due to an excessively high reaction temperature, which may lead to more chlorine-containing impurities (such as 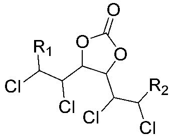 ), but also mitigates a risk of a reduced reaction rate, a prolonged reaction duration, and a decreased production efficiency caused by an excessively low reaction temperature. Therefore, a favorable balance between the reaction efficiency and the purity of the final product can be realized.

In some specific embodiments of the present disclosure, in the step (2), a molar ratio of the compound 1 to the thionyl chloride may be 1:(2 to 3), which, for example, may be 1:2, 1:2.2, 1:2.5, 1:2.8, or 1:3. An addition duration of the thionyl chloride may range from 0.5 h to 2 h, which, for example, may be 0.5 h, 0.8 h, 1 h, 1.5 h, or 2 h. Controlling an addition amount of the thionyl chloride within the specified range not only reduces a risk of a reduced conversion rate of the hexahydric alcohol and the presence of unreacted hydroxyl groups due to relatively insufficient thionyl chloride, which can easily lead to decreased purity and yield of the compound 2 and the target product represented by the Formula I, but also reduces a risk of increased chlorine-containing impurities that may arise from excessive use of the thionyl chloride. Further, by maintaining the addition duration of the thionyl chloride within the given range, an appropriate addition rate can be achieved, which can not only enhance the reaction rate but also reduce the risk of the increased chlorine-containing impurities caused by excessive addition rate of the thionyl chloride. Therefore, both the reaction rate and product quality are favorably balanced, leading to improved purity of the compound 2 and the final product, and a reduced impurity content. The condensation reaction is performed at a duration ranging from 1 hour to 4 hours. This duration is a duration from a start of thionyl chloride addition to completion of the condensation reaction. This duration may be adaptively adjusted according to the addition duration of the thionyl chloride, ensuring that the reaction continues for a certain period after completion of thionyl chloride addition. In this way, the condensation reaction can be completed thoroughly.

In some specific embodiments of the present disclosure, in the step (2), the solvent may include, but is not limited to, one or more of an ether solvent, N,N-dimethylformamide (DMF), N,N-dimethylacetamide (DMAC), tetrahydrofuran, or an ester solvent.

In some specific embodiments of the present disclosure, in the step (3), the oxidizing agent is provided as an aqueous solution. Subsequent to reacting the compound 2 with the oxidizing agent, the method further includes: performing a heating treatment on a reaction product to remove water.

In some specific embodiments of the present disclosure, in the step (3), after completion of the oxidation reaction, the reaction product may be washed with water to remove inorganic salt. Specifically, an organic phase in the reaction product may be separated, and deionized water is added for extraction and layer separation. After repeating this process several times, the solvent is removed to obtain the compound represented by the Formula I. In some specific embodiments, solvent removal may be achieved by the reduced-pressure treatment. The pressure for the reduced-pressure treatment may range from -90 kPa to -80 kPa (i.e., 90 kPa to 80 kPa lower than the standard atmospheric pressure), which, for example, may be -90 kPa, -85 kPa, or -80 kPa. The temperature for the reduced-pressure treatment may range from 30°C to 60°C, such as 35°C, 40°C, 45°C, 50°C, or 55°C. Therefore, further removing a solvent component can be facilitated.

In some specific embodiments of the present disclosure, in the step (3), a specific type of the oxidizing agent is not particularly limited, which can be selected flexibly by those skilled in the art as desired. For example, the oxidizing agent may include, but is not limited to, one or more of hydrogen peroxide, sodium hypochlorite, or sodium periodate. For example, the oxidizing agent may be sodium hypochlorite. For another example, the oxidizing agent may be hydrogen peroxide. Selecting the hydrogen peroxide can not only further reduce chlorine-containing impurities that may be introduced into the final product, but also enable the generated water to be removed by the heating treatment.

In some specific embodiments of the present disclosure, in the step (3), the oxidizing agent may include the sodium hypochlorite and/or the sodium periodate. A molar ratio of the compound 2 to the oxidizing agent may be 1:(2 to 2.5), such as 1/2, 1/2.1, 1/2.2, 1/2.3, 1/2.4, or 1/2.5. Controlling a content of the oxidizing agent within the above range is not only beneficial for fully oxidizing the compound 2 and improving the yield and the purity of the target product represented by the Formula I, but also can reduce chlorine-containing impurities and/or inorganic salt impurities that may be introduced when the oxidizing agent is used in an excessive amount.

In some specific embodiments of the present disclosure, in the step (3), the oxidizing agent may include the sodium hypochlorite and/or the sodium periodate. The oxidizing agent may be provided as the aqueous solution. Before the compound 2 reacts with the oxidizing agent, the condensation reaction product is dissolved in a non-aqueous solvent, and then the oxidizing agent is added for the oxidation reaction. After the oxidation reaction, the process further includes: (4-1) performing the heating treatment on the reaction product to remove water; (4-2) filtering to remove the inorganic salt that precipitates out after water removal. Therefore, not only can water and inorganic salt be removed from the final product, for example a content of the sodium hypochlorite or the sodium periodate can be reduced to several tens or even several ppm, but also a loss of the target product caused by the water washing process can be avoided.

In a third aspect, the present disclosure provides a polycyclic compound prepared by the above method for preparing the polycyclic compound. The prepared polycyclic compound includes a compound represented by Formula I and a chlorine-containing organic compound. A content of the compound represented by the Formula I is greater than or equal to 99 wt%. A total content of the chlorine-containing organic compound is less than or equal to 300 ppm. The characteristics and effects described for the above polycyclic compound preparation method are also applicable to this polycyclic compound, and thus details thereof will be omitted here.

In a fourth aspect, the present disclosure provides use of the polycyclic compound according to the above or the above method for preparing the polycyclic compound in the field of electrolyte and battery. Characteristics and effects described for the above polycyclic compound and the above method for preparing the polycyclic compound are also applicable to this use, and thus details thereof will be omitted here.

In a fifth aspect, the present disclosure provides an electrolyte. The electrolyte includes an electrolyte additive. The electrolyte additive includes the polycyclic compound according to the above or the polycyclic compound prepared by the method according to the above. It should be noted that the characteristics and effects described for the above polycyclic compound and the above method for preparing the polycyclic compound are also applicable to this electrolyte, and thus details thereof will be omitted here. In general, using this electrolyte in the battery can well improve the electrochemical performance of the battery.

Typically, the electrolyte further includes an electrolyte salt and an organic solvent. Optionally, the electrolyte may be used in the lithium battery. Taking the lithium battery as an example, the electrolyte salt may be a lithium salt. The organic solvent is a main component of the electrolyte and an important carrier for ion transmission. The organic solvent may have a high solubility for the lithium salt, enabling the electrolyte to have high ionic conductivity. After the lithium salt dissolves in the organic solvent, lithium ions may be released. The lithium ions may form a solvated structure with the organic solvent, which is conducive to rapid migration of the lithium ions.

In some specific embodiments of the present disclosure, based on a total mass of the electrolyte, a content of the electrolyte additive may range from 0.1 wt% to 5 wt%, such as 0.5 wt%, 1 wt%, 1.5 wt%, 2 wt%, 2.5 wt%, 3 wt%, 3.5 wt%, 4 wt%, 4.5 wt%, or 5 wt%. Therefore, when the electrolyte is used in the battery, a good effect on improving the electrochemical performance of the battery can be realized.

In some specific embodiments of the present disclosure, the organic solvent may include a carbonate solvent and/or a carboxylate solvent. Therefore, dispersion uniformity and ionic conductivity of the electrolyte can be improved.

In some specific embodiments of the present disclosure, the organic solvent may include at least one of ethylene carbonate, propylene carbonate, dimethyl carbonate, diethyl carbonate, ethyl methyl carbonate, γ-butyrolactone, methyl acetate, ethyl acetate, propyl acetate, butyl acetate, ethyl propionate, propyl propionate, or butyl propionate. The organic solvent within the above range can enable the electrolyte to have the high ionic conductivity after dissolving the lithium salt, which is further conducive to improving the electrochemical performance of the battery.

In some specific embodiments of the present disclosure, composition of the organic solvent may be flexibly adjusted according to actual conditions such as a battery system. For example, as some specific examples, the organic solvent may include ethylene carbonate (EC), ethyl methyl carbonate (EMC), and dimethyl carbonate (DMC) in a mass ratio of 1:(0.5 to 2):(0.5 to 2). This organic solvent may be suitable for a battery system using a layered transition metal oxide and the like as a positive electrode active material (for example, a positive electrode active material with a chemical formula of LiₐNi_{b}Co_{c}M1_{d}M2ₑO_{f}R_{g}, where 1≤a≤1.2, 0.6≤b≤1, 0≤c≤1, 0≤d≤1, 0≤e≤0.2, b+c+d+e=1, 1≤f≤2, 0≤g≤1, f+g=2; M1 includes Mn and/or Al, M2 includes at least one of Zr, Zn, Cu, Cr, Mg, Fe, V, Ti, Sr, Sb, Y, W, or Nb, and R includes at least one of N, F, S, or Cl).

In some specific embodiments of the present disclosure, the lithium salt may include at least one of lithium hexafluorophosphate (LiPF₆), lithium bis(fluorosulfonyl)imide (LiFSI), lithium tetrafluoroborate (LiBF₄), or lithium bis(trifluoromethanesulfonyl)imide (C₂F₆LiNO₄S₂).

In some specific embodiments of the present disclosure, a molar concentration of the lithium salt in the electrolyte may range from 0.8 mol/L to 2 mol/L, which, for example, may be 0.8 mol/L, 0.9 mol/L, 1.0 mol/L, 1.1 mol/L, 1.2 mol/L, 1.3 mol/L, 1.4 mol/L, 1.5 mol/L, 1.6 mol/L, 1.7 mol/L, 1.8 mol/L, 1.9 mol/L, or 2 mol/L. A cost of the lithium salt accounts for a relatively high proportion of a total electrolyte cost. Controlling the concentration of the lithium salt in the electrolyte within the above range is not only beneficial for full dissolution of the lithium salt in the organic solvent, but also helps the electrolyte to have both the high ionic conductivity and a low manufacturing cost.

In some specific embodiments of the present disclosure, in addition to the above components, the electrolyte may optionally include a small amount of other conventional additives or auxiliaries that can improve certain properties of the battery. These additives or auxiliaries may be selected by those skilled in the art as desired, and thus details thereof will be omitted here.

In a sixth aspect, the present disclosure provides a battery. The battery includes the above electrolyte. It should be noted that characteristics and effects described for the above electrolyte are also applicable to this battery, and will not be repeated here. In general, this battery has good electrochemical performance. In addition, it should be noted that a type of battery is not particularly limited, which may be flexibly selected by those skilled in the art as desired. For example, the battery may be a secondary battery.

Typically, the battery includes a positive electrode plate, a negative electrode plate, an electrolyte, and a separator. During charging and discharging of the battery, active ions intercalate and deintercalate back and forth between the positive electrode plate and the negative electrode plate. The electrolyte may serve to conduct ions between the positive electrode plate and the negative electrode plate. The separator is disposed between the positive electrode plate and the negative electrode plate. The separator mainly functions to prevent a short circuit between a positive electrode and a negative electrode, while making the ions pass through.

In some embodiments of the present disclosure, the positive electrode plate may include a positive electrode current collector and a positive electrode active material layer disposed on a surface of the positive electrode current collector. The positive electrode active material layer includes a positive electrode active material, a conductive agent, and a binder. The positive electrode current collector may include a metal foil or a composite positive electrode current collector. For example, the metal foil may be an aluminum foil. The composite positive electrode current collector may include a polymer material substrate and a metal layer formed on at least one surface of the polymer material substrate. For example, a composite positive electrode current collector may be formed by forming a metal material (aluminum, aluminum alloy, nickel, nickel alloy, etc.) at a polymer material substrate (such as polypropylene (PP), polyethylene terephthalate (PET), polybutylene terephthalate (PBT), etc.). The positive electrode active material may include the above positive electrode active material or other positive electrode active materials commonly used in the art. The conductive agent and the binder may be conventional materials in the art.

In some embodiments of the present disclosure, the negative electrode plate may include a negative electrode current collector and a negative electrode active material layer disposed on a surface of the negative electrode current collector. The negative electrode active material layer may include a negative electrode active material, the conductive agent, and the binder. The negative electrode current collector may be the metal foil or a composite current collector. For example, the metal foil may be a copper foil. The composite current collector may include the polymer material substrate and the metal layer formed on the at least one surface of the polymer material substrate. For example, the composite current collector may be formed by forming the metal material (copper, copper alloy, nickel, nickel alloy, etc.) at the polymer material substrate (such as polypropylene (PP), polyethylene terephthalate (PET), polybutylene terephthalate (PBT), etc.). The negative electrode active material may include a negative electrode active material commonly used in the art (for example, may include, but is not limited to, at least one of graphite, a silicon-carbon composite material, a silicon material, lithium metal, lithium titanate, or other commonly used negative electrode active materials). The conductive agent and the binder may be conventional materials in the art.

In some embodiments of the present disclosure, the separator may be a separator known in the art that can be used in the battery and is stable to the electrolyte used. A material of the separator may include, but is not limited to, at least one of polyolefin, aromatic polyamide, polytetrafluoroethylene, or polyethersulfone. For example, the separator may be a polyethylene separator, a polypropylene separator, a PE ceramic-coated separator, etc., which may be flexibly selected as desired.

The embodiments of the present disclosure are described in detail below. It should be noted that the embodiments described below are illustrative, which are only intended to explain the present disclosure, and thus should not be construed as limiting the present disclosure. In addition, unless otherwise specified, all reagents used in the following examples are commercially available or can be synthesized according to the methods described herein or by known methods, and reaction conditions not otherwise specified are readily obtainable by those skilled in the art.

### Example 1

### 1. Preparation of polycyclic compound:

(i) Mannitol, dimethyl carbonate, methanol, and sodium hydroxide were put into a reaction kettle. The reaction was performed at 1 MPa and 75°C for 1 hour. Low-boiling materials were removed under a reduced pressure of (-85±5) kPa at 50°C to obtain a dry product mainly composed of a compound 1. A structural formula of the compound 1 is 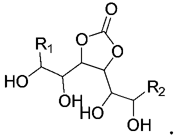 In this example, the structural formula is 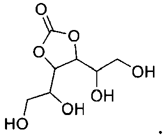
(ii) The dry product was taken and dissolved in dichloromethane (a mass ratio of the dichloromethane to the dry product was 5:2). At the room temperature, thionyl chloride was added dropwise to the dry product solution with an addition duration of 1.5 hours, followed by stirring for 3 hours. A molar ratio of 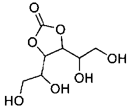 in the dry product to the thionyl chloride was 1:2.1. A reflux deacidification treatment was performed at 40°C for 30 minutes to obtain the reaction product. The reaction product contains the compound 2. A structural formula of the compound 2 is 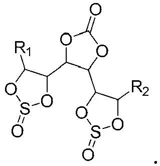 In this example, the structural formula is 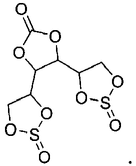
(iii) A 12% sodium hypochlorite aqueous solution was added to the reaction product of the step (2) for the oxidation reaction. Layer separation was performed, and the organic phase was taken. Washing with deionized water, extraction, and layer separation were performed, which were repeated for 3 times. The solvent was removed under the reduced pressure of (-85±5) kPa at 30°C to obtain the final product 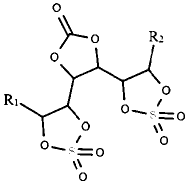 represented by the Formula I. In this example, the final product is 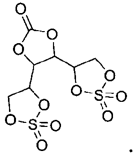 A molar ratio of the sodium hypochlorite to the compound 2 was 2:1.

### 2. Preparation of positive electrode plate

A positive electrode active material LiNi_{0.5}Co_{0.2}Mn_{0.3}O₂ (NCM523), a conductive agent Super P (conductive carbon black), and a binder polyvinylidene fluoride (PVDF) in a weight ratio of 96.8:2:1.2 were mixed uniformly, followed by addition of N-methylpyrrolidone (NMP). Stirring was performed using a vacuum mixer until the mixed system formed a positive electrode slurry with uniform fluidity. The positive electrode slurry was evenly coated on a positive electrode current collector aluminum foil, with a coating loading of 33 g/m². After drying at 85°C, cold pressing was performed, followed by edge trimming, plate cutting, and slitting. After slitting, drying was performed at 85°C under a vacuum condition for 4 hours. A tab was welded to obtain the positive electrode plate.

### 3. Preparation of negative electrode plate

A negative electrode active material graphite, the conductive agent Super P (conductive carbon black), a thickener CMC (sodium carboxymethyl cellulose), and a binder styrene-butadiene rubber in a weight ratio of 95:1.5:1.5:2 were mixed, followed by addition of the deionized water. A negative electrode slurry was obtained using the vacuum mixer. The negative electrode slurry was evenly coated on a negative electrode current collector copper foil, with a coating loading of 20.1 g/m². After drying at 85°C, the cold pressing was performed, followed by edge trimming, plate cutting, and slitting. After slitting, drying was performed at 85°C under the vacuum condition for 4 hours. The tab was welded to obtain the negative electrode plate.

### 4. Preparation of electrolyte

In an argon-filled glove box (water<10 ppm and oxygen<1 ppm), the electrolyte was prepared using ethylene carbonate (EC) and ethyl methyl carbonate (EMC) in a mass ratio of EC:EMC=3:7 as the organic solvent, lithium hexafluorophosphate (LiPF₆) at a concentration of 12.5% as the lithium salt, ethylene sulfate (DTD) as a conventional additive, and the final product obtained in the step 1 as the electrolyte additive, with the lithium salt concentration adjusted to 1 mol/L and a mass proportion 3 wt% of the electrolyte additive in the electrolyte.

### 5. Preparation of separator

A polyethylene separator with a thickness of 8 µm was selected.

### 6. Preparation of lithium-ion battery

The prepared positive electrode plate, negative electrode plate, and separator were stacked in sequence. The separator was placed between the positive electrode plate and the negative electrode plate. After winding and flattening, the assembly was placed into an aluminum foil packaging bag. The assembly was baked under a vacuum condition at 75°C for 48 hours to obtain a battery cell. The above-mentioned electrolyte was injected into the battery cell in the glove box. The preparation of the lithium-ion battery was completed after sealing, formation, aging, and capacity grading.

### Example 2 to Example 15 and Comparative Example 1 to Comparative Example 6

Differences of Example 2 to Example 15 and Comparative Example 1 to Comparative Example 6 from Example 1 were detailed in Table 1-1, Table 1-2, Table 2-1, and Table 2-2.

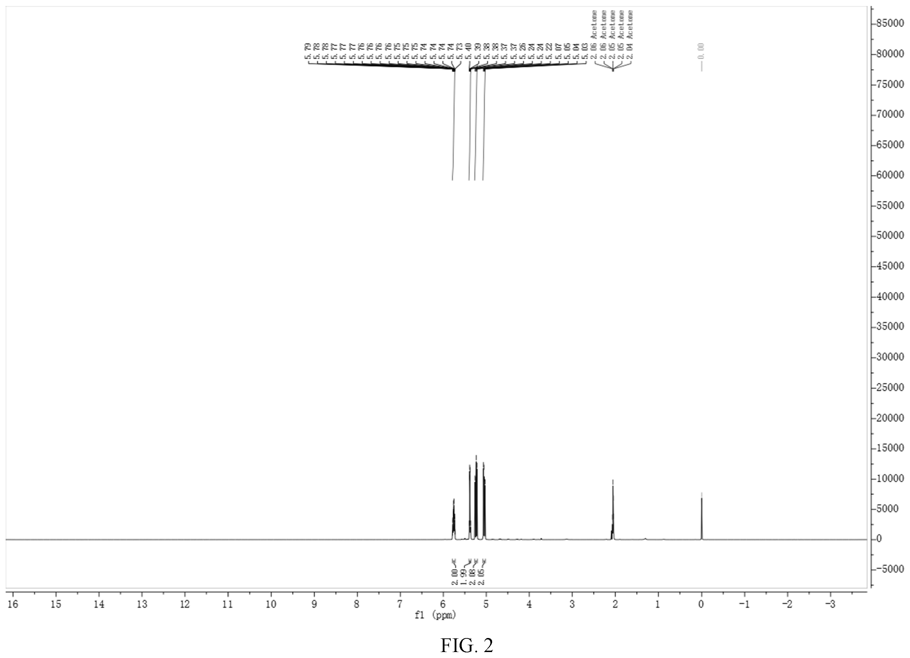

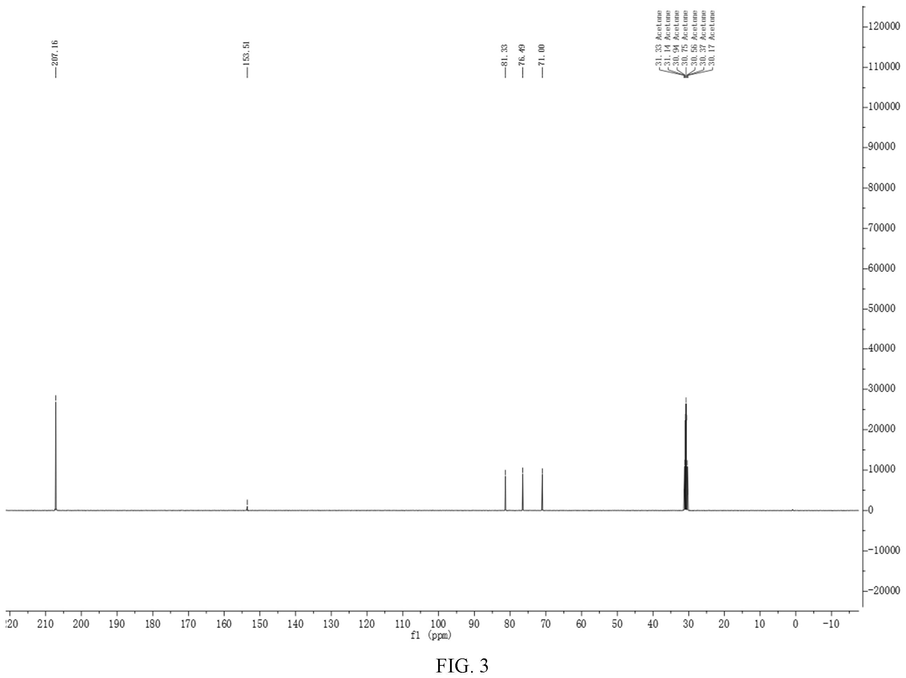

The structure, purity, and content of chlorine-containing organic impurities of the polycyclic compound obtained in Example 1 to Example 15 and Comparative Example 1 to Comparative Example 6, and cycling performance of the battery were tested. The test methods were as follows. The test results were shown in Table 2-1, Table 2-2, FIG. 2, and FIG. 3.
(1) A Nuclear Magnetic Resonance (NMR) spectroscopy was used to perform NMR test on the prepared polycyclic compound final product to determine a compound structure of the final product. A high-performance liquid chromatography was used to perform liquid chromatography test on the prepared polycyclic compound final product for quantitative analysis of the final product components. The high-performance liquid chromatography was used to analyze the content of the chlorine-containing organic compound.
(2) Cycling performance test: at the room temperature, the formed battery was charged to 4.2 V with a constant current of 1 C and a constant voltage, then charged at a constant voltage of 4.2 V until a cut-off current reached 0.05 C, and discharged to 2.75 V at 1 C to measure an initial discharge capacity of the battery. 1000 charge-discharge cycles were performed. A discharge capacity of the battery after 500 charge-discharge cycles was measured. A cycling capacity retention rate was calculated based on the formula: Cycling capacity retention rate (%)=(Discharge capacity at the 500th cycle/Discharge capacity at the 1st cycle)×100%.
(3) High-temperature storage test: the battery was charged to 4.2 V with a constant current of 1.0 C at 25°C, then charged at the constant voltage of 4.2 V until the cut-off current reached 0.05 C, and then discharged to 2.75 V with the constant current of 1.0 C. The discharge capacity was recorded as C1. At 25°C, the battery was charged to 4.2 V with the constant current of 1.0 C, then charged at the constant voltage of 4.2 V until the cut-off current reached 0.05 C. The battery was then transferred to 60°C for storage for 30 days, and then discharged to 2.75 V with the constant current of 1.0 C. The discharge capacity was recorded as C2. The capacity retention rate after 30 days of storage at 60°C = C2/C1x100%.

### Example 16

The preparation of the polycyclic compound was completely the same as that in Example 1. Example 16 mainly differed from Example 1 in the preparation of the negative electrode plate, the positive electrode plate, the electrolyte, and the lithium battery. Test conditions for relevant tests were also different. The details were as follows.

### 2. Preparation of positive electrode plate

The positive electrode active material LiCoO₂, the conductive agent Super P (conductive carbon black), and the binder polyvinylidene fluoride (PVDF) in a weight ratio of 96.8:2:1.2 were mixed uniformly, followed by addition of N-methylpyrrolidone (NMP). Stirring was performed using the vacuum mixer until the mixed system formed the positive electrode slurry with uniform fluidity. The positive electrode slurry was evenly coated on the positive electrode current collector aluminum foil, with a coating loading of 33 g/m². After drying at 85°C, the cold pressing was performed, followed by edge trimming, plate cutting, and slitting. After slitting, drying was performed at 85°C under the vacuum condition for 4 hours. The tab was welded to obtain the positive electrode plate.

### 3. Preparation of negative electrode plate

The negative electrode active material graphite, the conductive agent Super P (conductive carbon black), the thickener CMC (sodium carboxymethyl cellulose), and the binder styrene-butadiene rubber in a weight ratio of 95:1.5:1.5:2 were mixed, followed by addition of the deionized water. The negative electrode slurry was obtained using the vacuum mixer. The negative electrode slurry was evenly coated on the negative electrode current collector copper foil, with a coating loading of 20.1 g/m². After drying at 85°C, the cold pressing was performed, followed by edge trimming, plate cutting, and slitting. After slitting, drying was performed at 85°C under the vacuum condition for 4 hours. The tab was welded to obtain the negative electrode plate.

### 4. Preparation of electrolyte

In the argon-filled glove box (water<10 ppm and oxygen<1 ppm), the electrolyte was prepared using the ethylene carbonate (EC) and the ethyl methyl carbonate (EMC) in the mass ratio of EC:EMC=3:7 as the organic solvent, the lithium hexafluorophosphate (LiPF₆) at the concentration of 12.5% as the lithium salt, the ethylene sulfate (DTD) as the conventional additive, and the final product obtained in the step 1 as the electrolyte additive, with the lithium salt concentration adjusted to 1 mol/L and the mass proportion 3 wt% of the electrolyte additive in the electrolyte.

### 5. Preparation of separator

The polyethylene separator with the thickness of 8 µm was selected.

### 6. Preparation of lithium-ion battery

The prepared positive electrode plate, negative electrode plate, and separator were stacked in sequence. The separator was placed between the positive electrode plate and the negative electrode plate. After winding and flattening, the assembly was placed into the aluminum foil packaging bag. The assembly was baked under a vacuum condition at 75°C for 48 hours to obtain the battery cell. The above-mentioned electrolyte was injected into the battery cell in the glove box. The preparation of the lithium-ion battery was completed after sealing, formation, aging, and capacity grading.

The cycling performance of the battery obtained in Example 16 was tested. The test methods were as follows. The test results were shown in Table 2-2.
(2) Cycling performance test: at the room temperature, the formed battery was charged to 4.53 V with the constant current of 1 C and constant voltage, then charged at the constant voltage of 4.53 V until the cut-off current reached 0.05 C, and discharged to 2.75 V at 1 C to measure the initial discharge capacity of the battery. 1000 charge-discharge cycles were performed. The discharge capacity of the battery after 500 charge-discharge cycles was measured. The cycling capacity retention rate was calculated based on the formula: Cycling capacity retention rate (%)=(Discharge Capacity at the 500th Cycle/Discharge Capacity at the 1st Cycle)×100%.
(3) High-temperature storage test: the battery was charged to 4.53 V with a constant current of 1.0 C at 25°C, charged at the constant voltage of 4.53 V until the cut-off current reached 0.05 C, and then discharged to 2.75 V with the constant current of 1.0 C. The discharge capacity was recorded as C1. At 25°C, the battery was charged to 4.53 V with the constant current of 1.0 C, charged at the constant voltage of 4.53 V until the cut-off current reached 0.05 C, and then the battery was transferred to 60°C for storage for 30 days. Then, the battery was discharged to 2.75 V with the constant current of 1.0 C. The discharge capacity was recorded as C2. The capacity retention rate after 30 days of storage at 60°C = C2/C1x100%.

### Example 17

Example 17 mainly differed from Example 1 in the preparation of the negative electrode plate, the positive electrode plate, the electrolyte, and the lithium battery. Also, the test conditions for the relevant tests were also different. The details were as follows.

### 2. Preparation of positive electrode plate

A positive electrode active material LiFePO₄, the conductive agent Super P (conductive carbon black), a conductive agent CNT, and the binder polyvinylidene fluoride (PVDF) in a weight ratio of 96.5:1:0.5:2 were mixed uniformly, followed by addition of N-methylpyrrolidone (NMP). Stirring was performed using the vacuum mixer until the mixed system formed the positive electrode slurry with uniform fluidity. The positive electrode slurry was evenly coated on the positive electrode current collector aluminum foil, with the coating loading of 33 g/m². After drying at 85°C, the cold pressing was performed, followed by edge trimming, plate cutting, and slitting. After slitting, drying was performed at 85°C under the vacuum condition for 4 hours. The tab was welded to obtain the positive electrode plate.

### 3. Preparation of negative electrode plate

The negative electrode active material graphite, the conductive agent Super P (conductive carbon black), the thickener CMC (sodium carboxymethyl cellulose), and the binder styrene-butadiene rubber in a weight ratio of 95:1.5:1.5:2 were mixed, followed by addition of the deionized water. The negative electrode slurry was obtained using the vacuum mixer. The negative electrode slurry was evenly coated on the negative electrode current collector copper foil, with the coating loading of 20.1 g/m². After drying at 85°C, the cold pressing was performed, followed by edge trimming, plate cutting, and slitting. After slitting, drying was performed at 85°C under the vacuum condition for 4 hours. The tab was welded to obtain the negative electrode plate.

### 4. Preparation of electrolyte

In the argon-filled glove box (water<10 ppm and oxygen<1 ppm), the electrolyte was prepared using the ethylene carbonate (EC) and the ethyl methyl carbonate (EMC) in the mass ratio of EC:EMC=3:7 as the organic solvent, the lithium hexafluorophosphate (LiPF₆) at the concentration of 12.5% as the lithium salt, the ethylene sulfate (DTD) and vinylene carbonate (VC) as the conventional additive, and the final product obtained in the step 1 as the electrolyte additive, with the lithium salt concentration adjusted to 1 mol/L and the mass proportion 5 wt% of the electrolyte additive in the electrolyte.

5. Preparation of separator

The polyethylene separator with the thickness of 8 µm was selected.

### 6. Preparation of lithium-ion battery

The prepared positive electrode plate, negative electrode plate, and separator were stacked in sequence. The separator was placed between the positive electrode plate and the negative electrode plate. After winding and flattening, the assembly was placed into the aluminum foil packaging bag. The assembly was baked under a vacuum condition at 75°C for 48 hours to obtain the battery cell. The above-mentioned electrolyte was injected into the battery cell in the glove box. The preparation of the lithium-ion battery was completed after sealing, formation, aging, and capacity grading.

The cycling performance of the battery obtained in Example 16 was tested. The test methods were as follows. The test results were shown in Table 2-2.
(2) Cycling performance test: at the room temperature, the formed battery was charged to 3.65 V with the constant current of 1 C and constant voltage, then charged at the constant voltage of 3.65 V until the cut-off current reached 0.05 C, and discharged to 2.0 V at 1 C to measure the initial discharge capacity of the battery. 2000 charge-discharge cycles were performed. A discharge capacity of the battery after 1000 charge-discharge cycles was measured. The cycling capacity retention rate was calculated based on the formula: Cycling capacity retention rate (%)=(Discharge Capacity at the 1000th Cycle/Discharge Capacity at the 1st Cycle)×100%.
(3) High-temperature storage test: the battery was charged to 3.65 V with the constant current of 1.0 C at 25°C, charged at the constant voltage of 3.65 V until the cut-off current reached 0.05 C, and then discharged to 2.0 V with the constant current of 1.0 C. The discharge capacity was recorded as C1. At 25°C, the battery was charged to 3.65 V with the constant current of 1.0 C, charged at the constant voltage of 3.65 V until the cut-off current reached 0.05 C, and then the battery was transferred to 60°C for storage for 30 days. Then, the battery was discharged to 2.0 V with the constant current of 1.0 C. The discharge capacity was recorded as C2. The capacity retention rate after 30 days of storage at 60°C = C2/C1x100%.

### Example 18 to Example 19

Differences between Examples 18 to 19 and Example 1 were detailed in Table 1-2, Table 2-2, and Table 2-3.

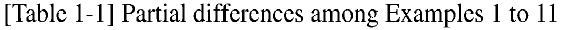

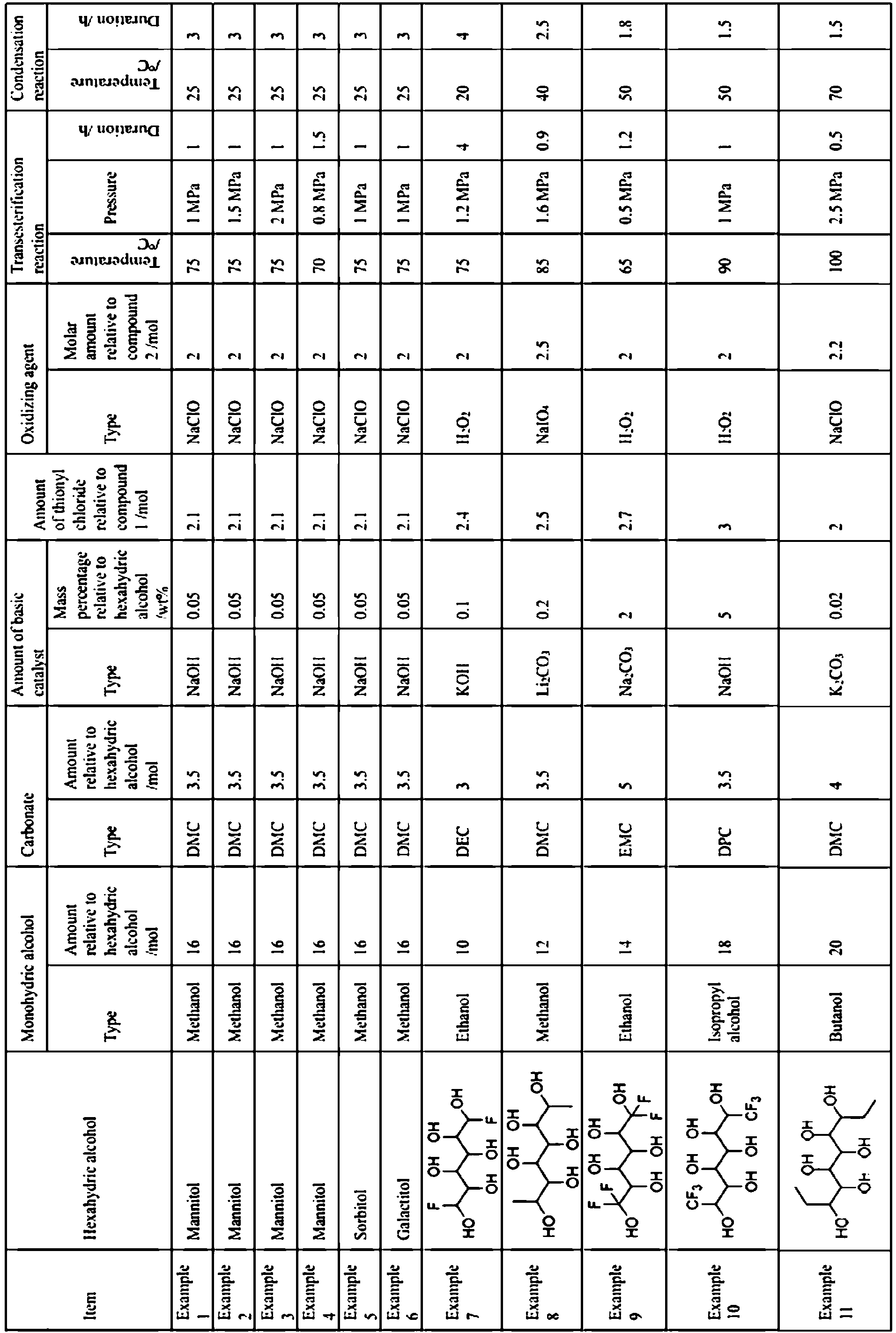

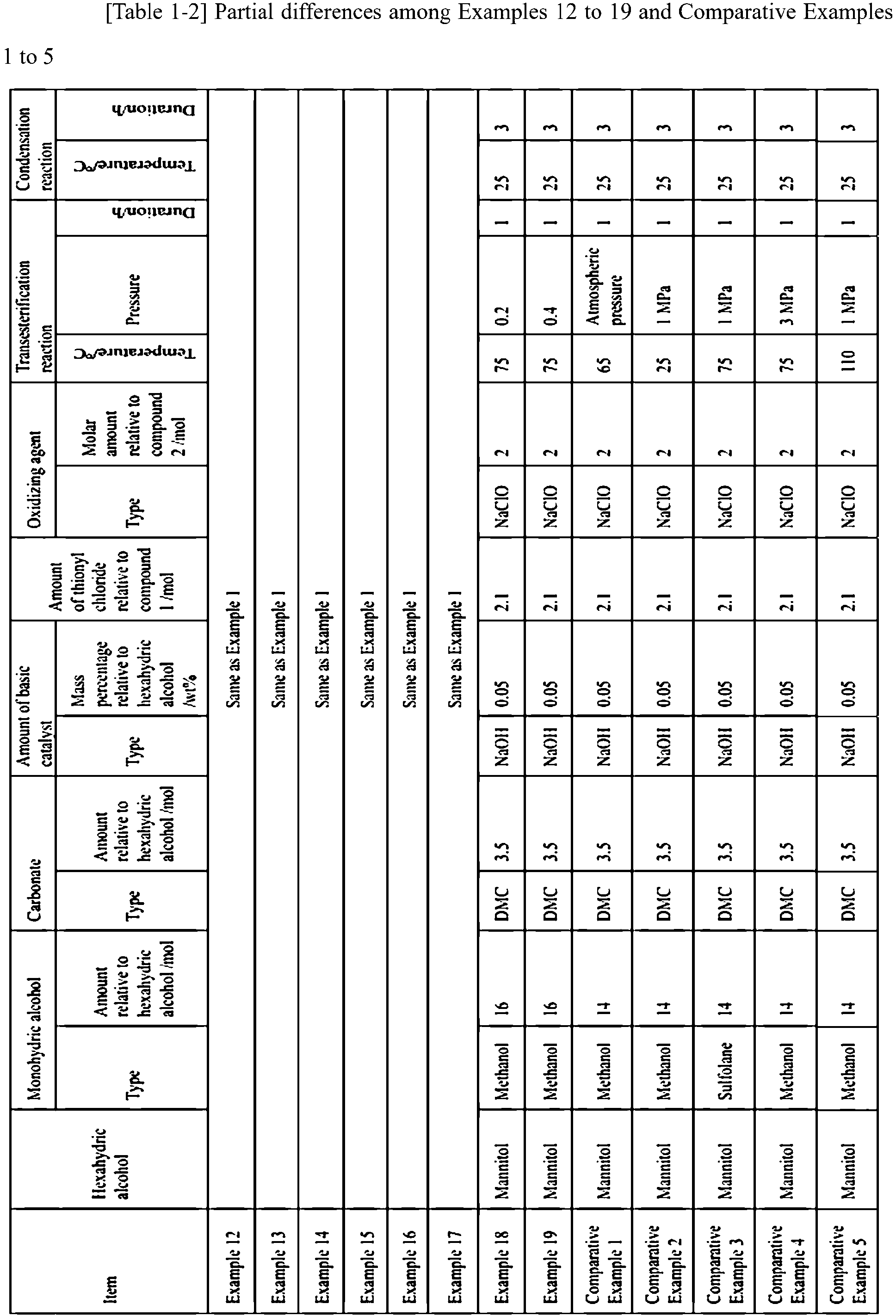

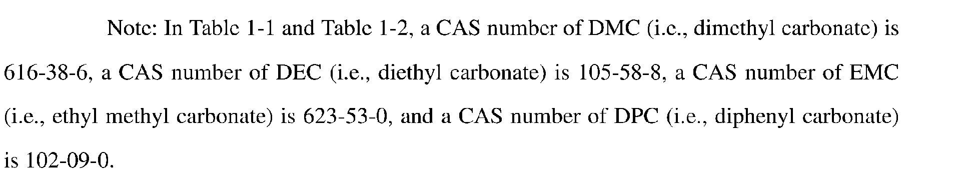

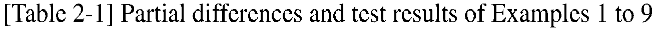

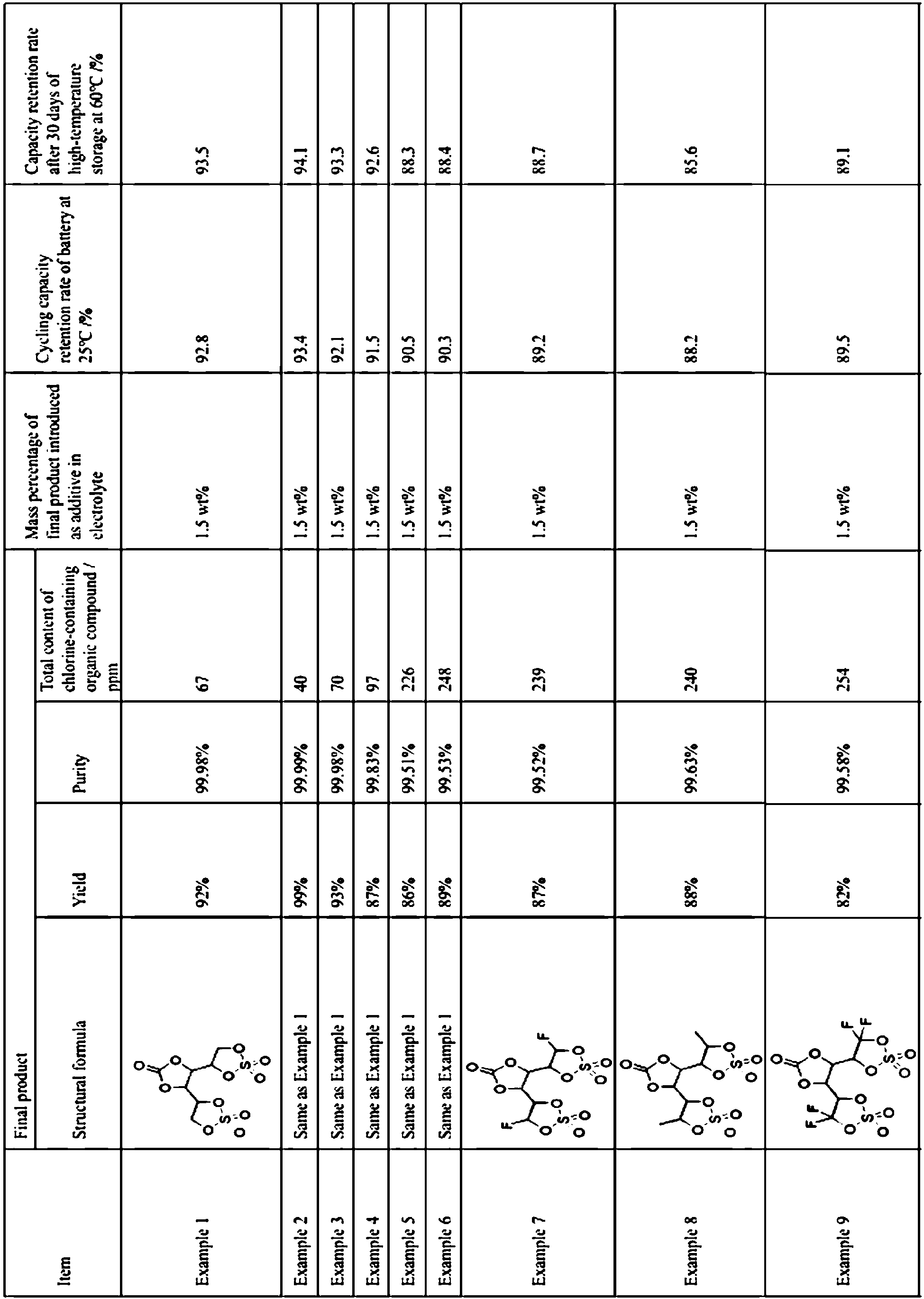

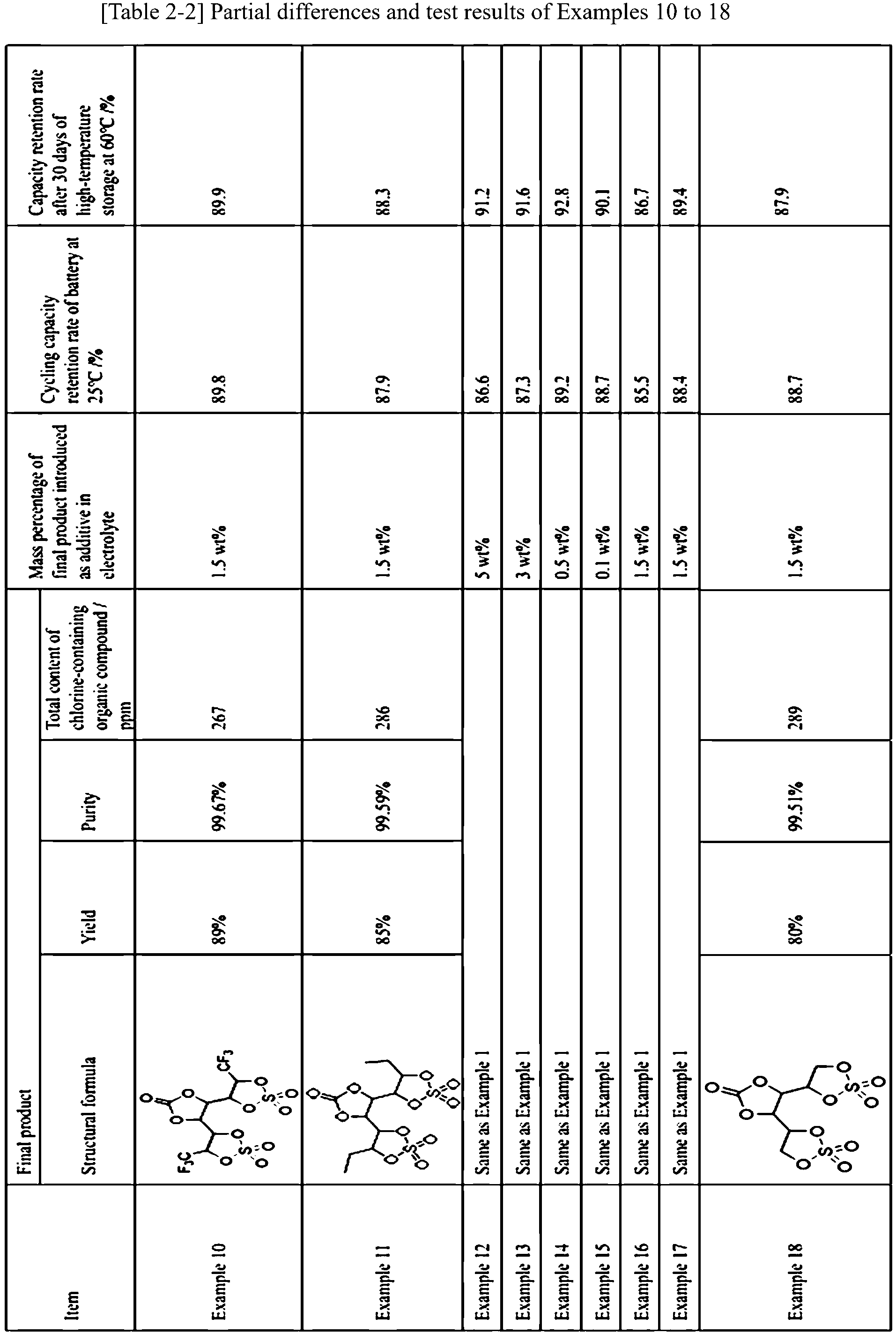

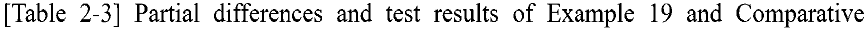

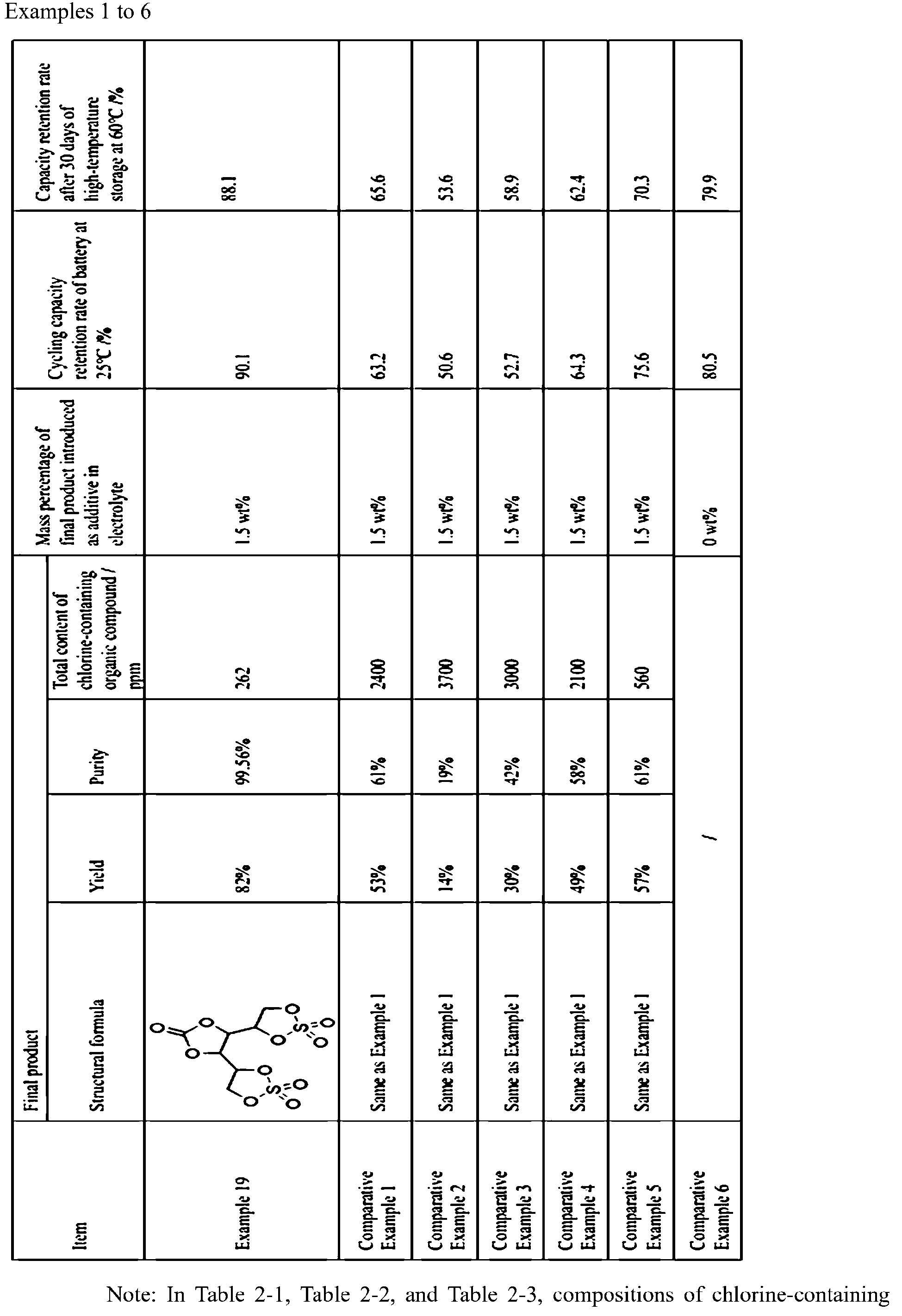

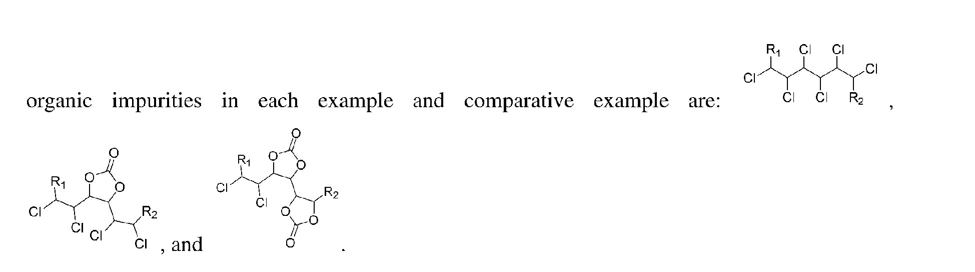

### Results and conclusions

I. It can be seen from Examples 1 to 19, Comparative Examples 1 to 6, Table 1-1, Table 1-2, Table 2-1, Table 2-2, and Table 2-3 that the final product prepared by the methods of the above examples of the present disclosure has higher purity and a lower total content of chlorine-containing organic impurities. The total content of the impurities is generally below 300 ppm. Taking Example 1 as an example, FIG. 2 and FIG. 3 show a proton nuclear magnetic resonance spectrum and a carbon nuclear magnetic resonance spectrum of the final product prepared in Example 1. The structural formula of the final product is shown in Table 2-1, with high purity and a total content of chlorine-containing organic impurities of 67 ppm. In addition, it can be seen from Examples 1 to 4 and Comparative Examples 1, 2, 4, and 5 that under the same condition, performing the transesterification reaction under specific heating and pressurization conditions can also improve the selectivity and yield of the target product, which can be further improved when appropriately increasing the pressure. It can be seen from Example 1 and Comparative example 3 that under the same condition, use of the monohydric alcohol can be highly effective in improving the yield, the purity, and reducing the content of chlorine-containing organic compounds. Further, it can also be seen from Examples 1 to 15 and Comparative Examples 1 to 6 that using the final product (i.e., the polycyclic compound) prepared in the above examples, with the purity of no less than 99% and the content of the chlorine-containing organic compounds below 300 ppm, as the electrolyte additive in the electrolyte is also beneficial to further improving the electrochemical performance of the battery.

In addition, it can be seen from Examples 1, 16, and 17 that when the final product (i.e., the polycyclic compound) prepared in the above examples, with the purity of no less than 99% and the content of the chlorine-containing organic compounds below 300 ppm, is used as the electrolyte additive in the electrolyte and applied to lithium batteries with different positive electrode active materials, the electrochemical performance of the battery can be significantly improved.

II. Taking the products obtained in Examples 6 and 2 as examples, the impurities in the samples were characterized.

### 1. Characterization of impurities in the product (Sample A) prepared in Example 6

Sample A was tested by liquid chromatography. At the same time, according to a retention time, samples of impurity peaks at different time periods were collected from a detector inlet section (a chromatographic column outlet section). Subsequently, the collected components were subjected to nitrogen blowing, rotary evaporation, and redissolution, and qualitative instruments such as LCMS, GCMS, and NMR were used to perform qualitative analysis on a substance structure of each component. Among multiple collected samples, 3 samples (hereinafter referred to as Sample 1, Sample 2, and Sample 3) conformed to the structures of 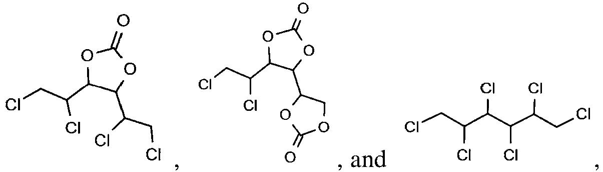 respectively. The qualitative analysis of specific impurities will be described in detail below.

### (1) Qualitative analysis of Sample 1

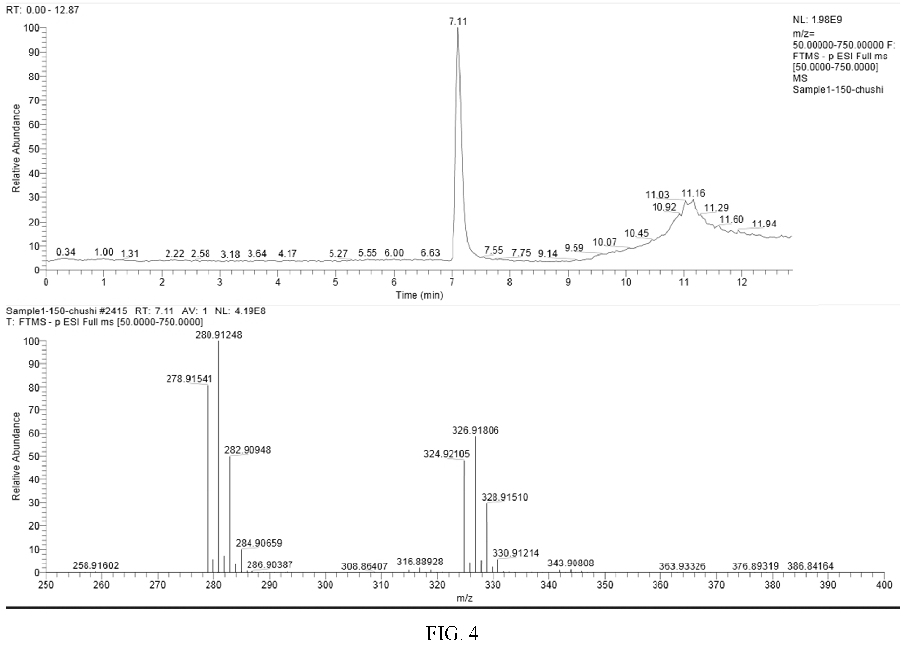

Sample 1 was separated by the liquid chromatography, enriched using the above collection method for the impurity samples , and then qualitatively analyzed by LCMS. Results were shown in FIG. 4.

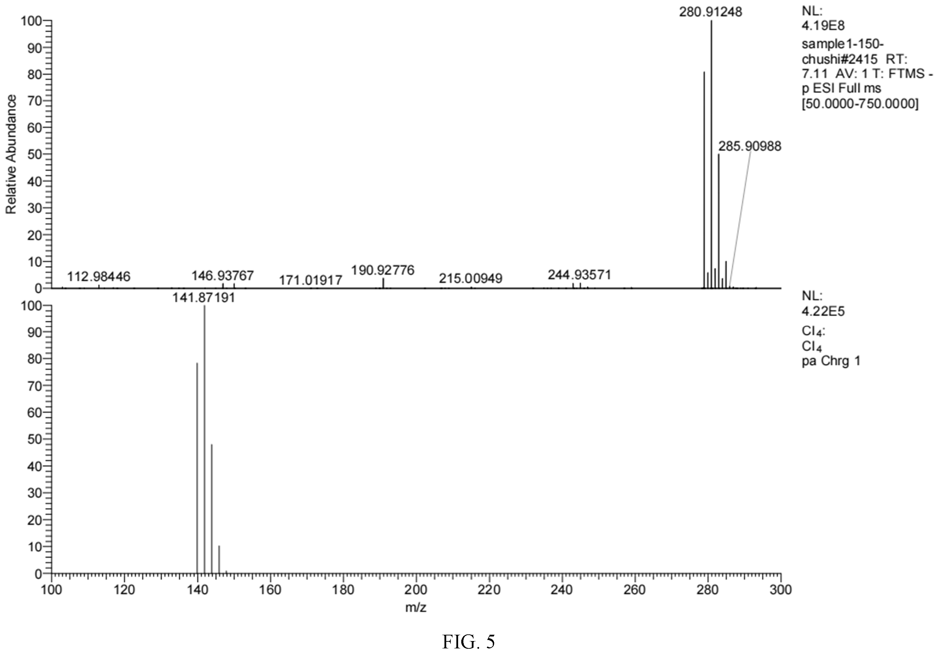

It can be seen from a mass spectrum bar graph of Sample 1 that Sample 1 contains M+2, M+4, M+6, and M+8 isotope peaks. A height ratio of each isotope peak in the sample is consistent with that in Cl₄ (FIG. 5). Therefore, it can be inferred that Sample 1 contains 4 Cl atoms. By a difference between m/z 324.92105 and m/z 278.91541 being 46.00564, it can be inferred that m/z 278.91541 is an [M-H]⁻ peak, and m/z 324.92105 is an [M+FA-H]⁻ peak. Based on background information of Sample 1, Sample 1 may contain C, O, Cl, and H. Therefore, a molecular formula of m/z 278.91541 is inferred to be C₇H₇Cl₄O₃, i.e., M is C₇H₈Cl₄O₃, with a degree of unsaturation RDB of 2. A specific structure of Sample 1 was further determined by NMR.

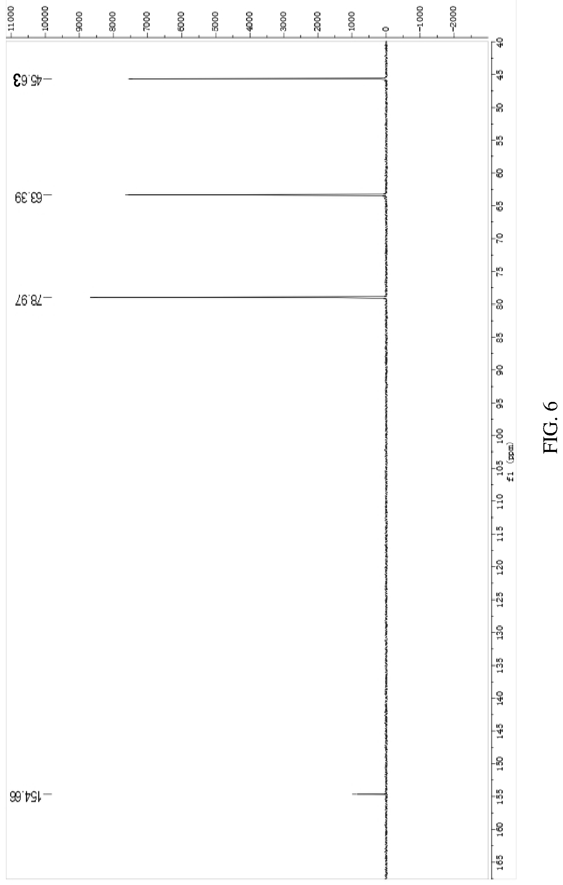

As can be seen from FIG. 6, Sample 1 has only 4 groups of C atoms with different chemical shifts. A molecular formula of C₇H₈Cl₄O₃ was inferred from LCMS, indicating that Sample 1 may have a symmetric structure. The signal at a chemical shift of 154 ppm corresponds to the -C=O carbon of the cyclic carbonate. The signal at a chemical shift of 78 ppm corresponds to the -CH- carbon bonded to chlorine. The signal at a chemical shift of 63 ppm corresponds to the -CH- carbon bonded to oxygen. The signal at a chemical shift of 45 ppm corresponds to the -CH₂- carbon bonded to a heteroatom or element with high electronegativity.

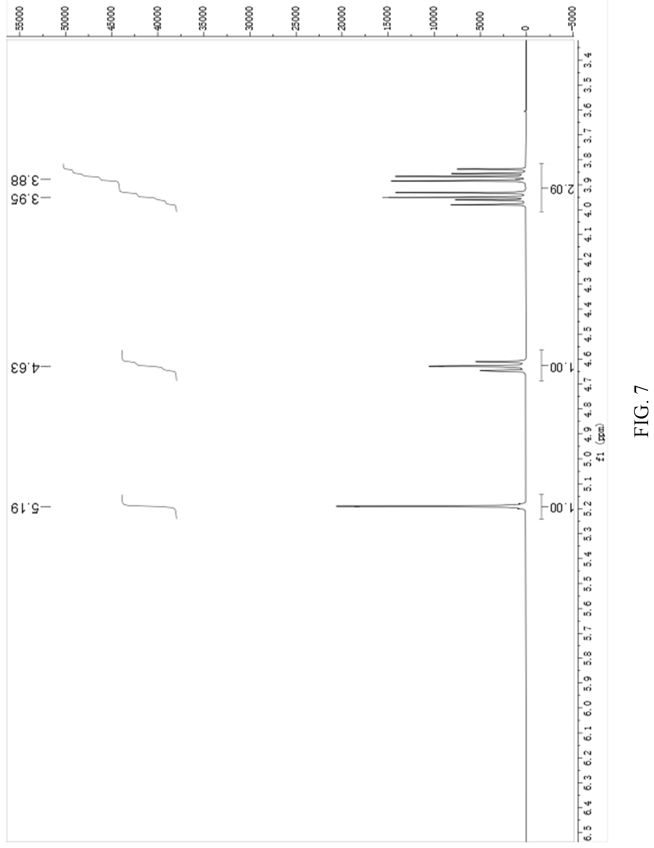

As can be seen from FIG. 7, Sample 1 has 3 groups of H atoms with different chemical shifts. A ratio of the three groups of H atoms is 1:1:2. Based on information from LCMS and C-NMR, a doublet at a chemical shift of 3.96 ppm is inferred to be the -CH₂- group bonded to the heteroatom, with 1 H atom on an adjacent carbon. A peak at a chemical shift of 4.63 ppm is inferred to be the -CH- group bonded to Cl or O. A peak at a chemical shift of 5.19 ppm is inferred to be the -CH- group, which contains an H atom on the carbon bonded to O in the cyclic carbonate.

Comprehensively considering information from LCMS, C-NMR, H-NMR, the reaction process, and the raw material, this substance structure is inferred to be

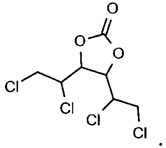

### (2) Qualitative analysis of Sample 2

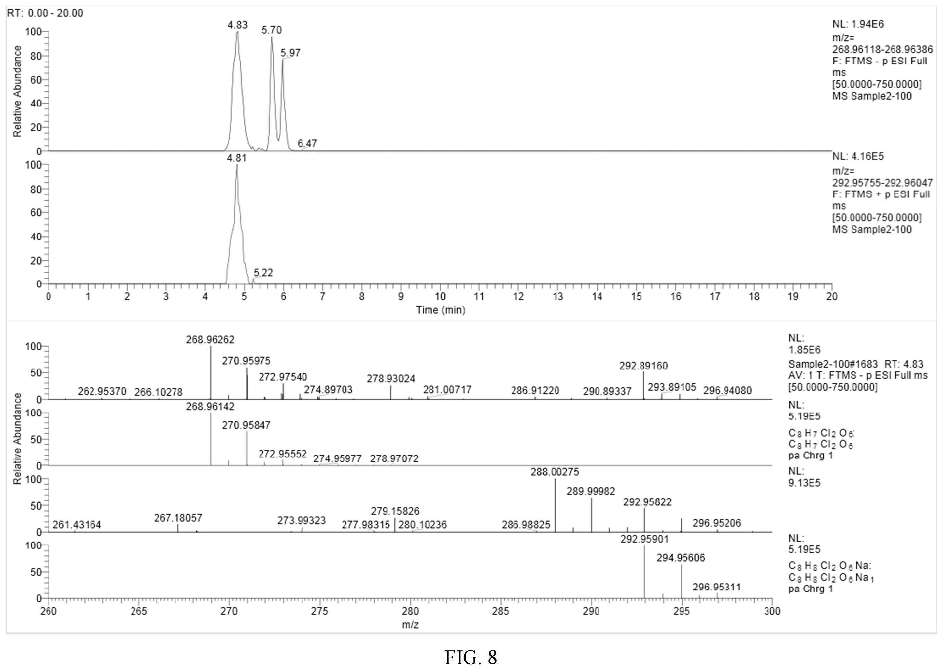

Sample 2 was separated by the liquid chromatography, enriched using the above collection method for the impurity samples, and then qualitatively analyzed by LCMS. Results were shown in FIG. 8.

It can be seen from a mass spectrum bar graph of Sample 2 that Sample 2 contains M+2 and M+4 isotope peaks, and a peak height thereof conforms to M:(M+2):(M+4)=9:6:1, indicating that Sample 2 contains 2 Cl atoms. A difference between m/z 268.96262 of a negative ion mode and m/z 292.95822 of a positive ion mode is 23.9956. Therefore, it can be inferred that m/z 268.96262 is the [M-H]⁻ peak, and m/z 292.95822 is an [M+Na]⁺ peak. Based on background information of Sample 2, Sample 2 may contain C, O, Cl, and H. Therefore, a molecular formula of m/z 268.96262 is inferred to be C₈H₇Cl₂O₆, i.e., M is C₈H₈Cl₂O₆, with the degree of unsaturation RDB of 4. A specific structure of Sample 2 was further determined by NMR.

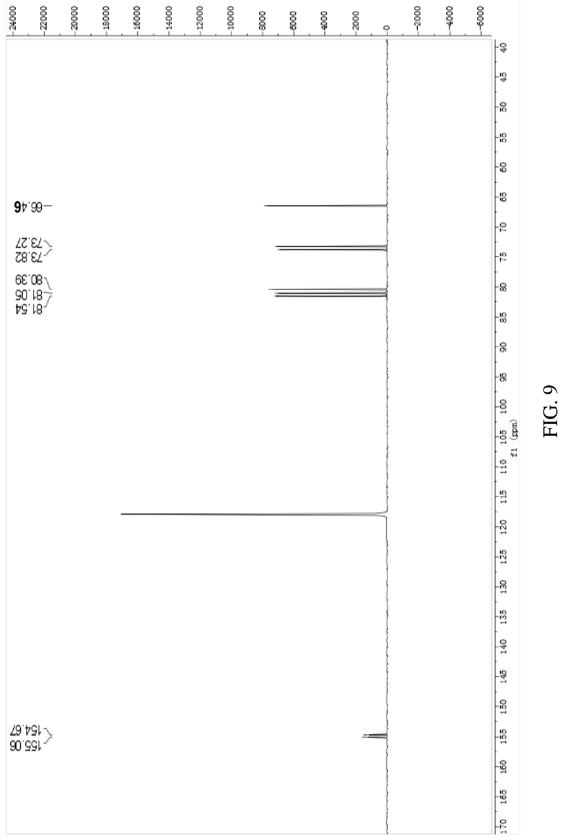

As can be seen from FIG. 9, Sample 2 has 8 groups of C atoms with different chemical shifts. The number of C atoms is consistent with the molecular formula C₈H₈Cl₂O₆ inferred by LCMS. Two peaks at chemical shifts of 154.0 ppm and 153.7 ppm correspond to the -C=O carbon on the two cyclic carbonates. The signal at a chemical shift of 65.6 ppm corresponds to the -CH₂- carbon bonded to O. The signal at a chemical shift of 72.7 ppm may correspond to the -CH₂- carbon bonded to Cl. The signals at chemical shifts of 73.1 ppm, 79.6 ppm, 80.8 ppm, and 80.7 ppm correspond to the -CH- carbon bonded to O or Cl. -CH- groups at chemical shifts of 80.8 ppm and 80.7 ppm may have adjacent carbon bonded to Cl with higher electronegativity, leading to chemical shifts thereof moving to a low field.

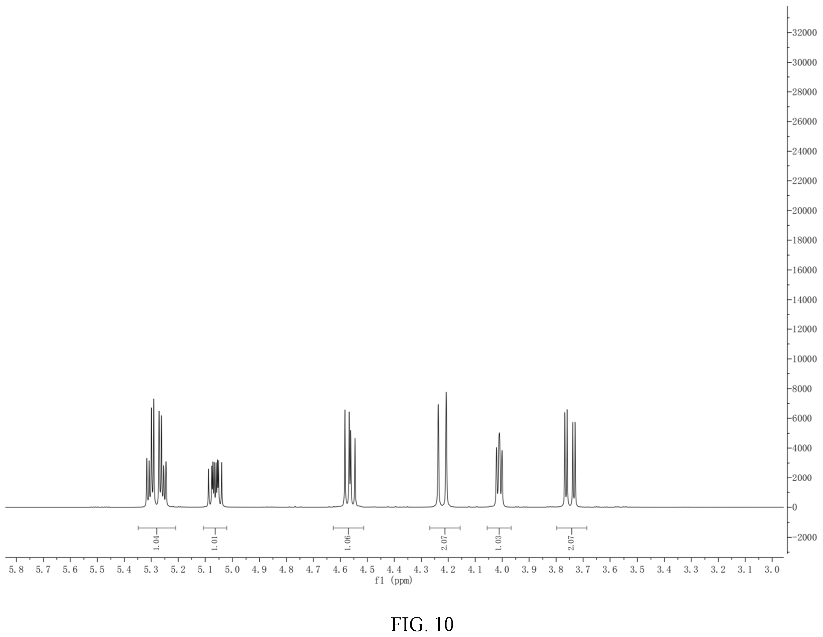

As can be seen from FIG. 10, Sample 2 has 6 groups of H atoms with different chemical shifts. A ratio of the six groups of H atoms is 1:1:1:2:1:2. Based on the information from LCMS and C-NMR, a peak at a chemical shift of 3.6 ppm is inferred to be two H atoms of the -CH₂- group bonded to Cl. Based on the number of a split peak, there is 1 H atom on the adjacent carbon. A peak at a chemical shift of 4.0 ppm is inferred to be the -CH- group bonded to Cl or O. Based on the number of the split peak being 6, it can be inferred that two adjacent carbon atoms have 2 H atoms and 1 H atom, respectively. A peak at a chemical shift of 4.25 ppm is inferred to be the -CH₂- group, which may be the -CH₂- group bonded to O in the cyclic carbonate. Based on the number of the split peak being 2, it can be inferred that there is 1 H atom on the adjacent carbon. A peak at a chemical shift of 4.55 ppm is inferred to be the -CH- group bonded to O or the cyclic carbonate. The number of the split peak indicates that there are 2 H atoms on the adjacent carbon. According to inference from C-NMR, Sample 2 may contain two cyclic carbonate structures. Therefore, two peaks at chemical shifts of 5.1 ppm and 5.25 ppm may be H atoms of the -CH- groups linking the two cyclic carbonates.

Comprehensively considering the information from LCMS, C-NMR, H-NMR, the reaction process, and the raw material, this substance structure is inferred to be

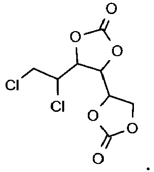

### (3) Qualitative analysis of Sample 3

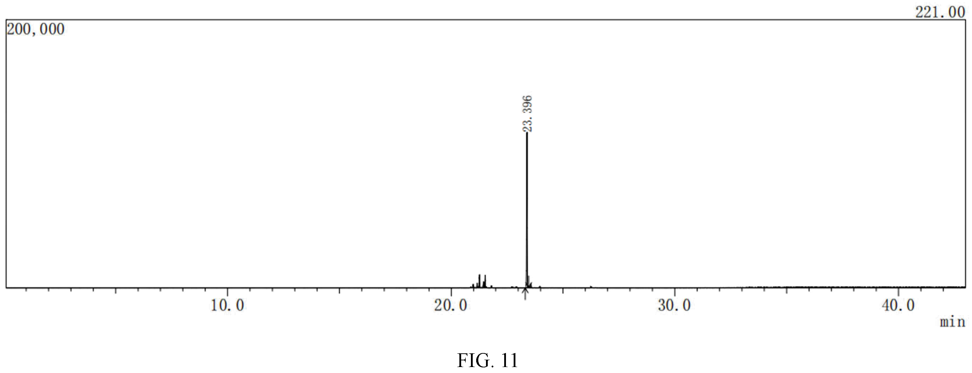

Sample 3 was separated by the liquid chromatography, enriched using the above collection method for the impurity samples, and then qualitatively analyzed by GCMS. Results were shown in FIG. 11.

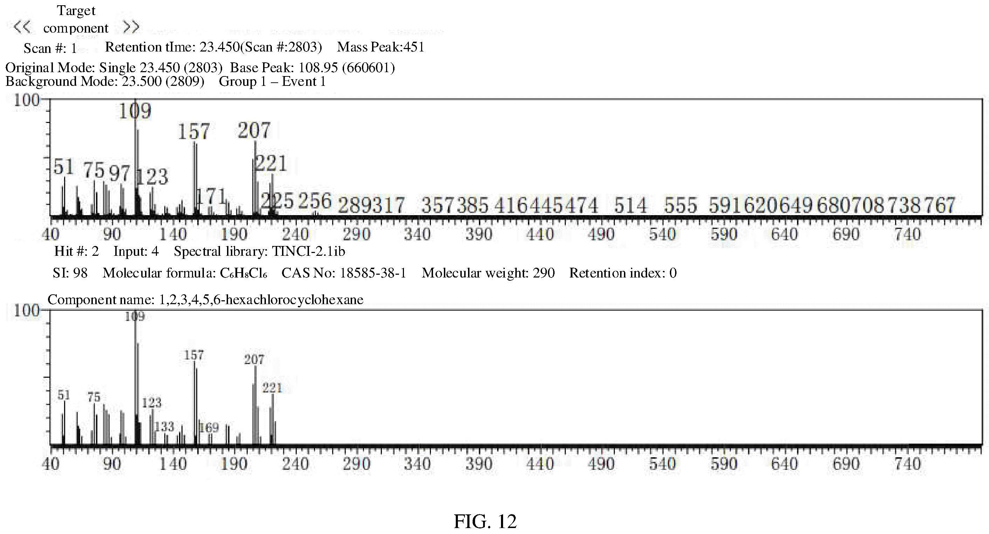

A substance with a retention time t=23.400 min was qualitatively analyzed by the mass spectral library (FIG. 12). A mass spectrum of this substance shows a similarity (SI) of 98% to a standard substance with CAS No. 18585-38-1 in the mass spectral library. Therefore, this substance is inferred to be

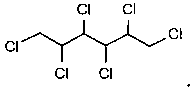

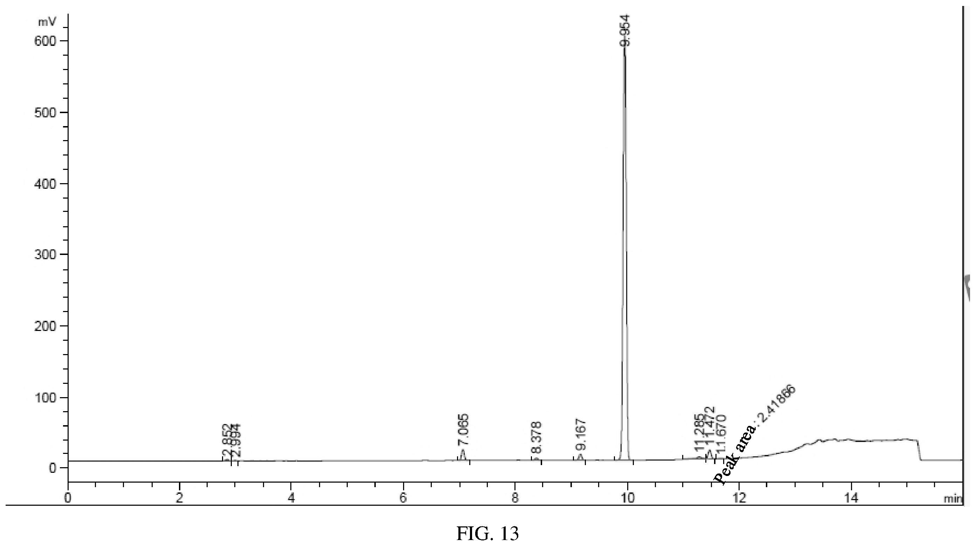

The liquid chromatogram of Sample A was shown in FIG. 13 below. A main peak in FIG. 13 is the target product. Based on qualitative analysis of impurity peaks at different time periods, a peak at 9.167 min is identified as Sample 2, a peak at 11.472 min is identified as Sample 1, and a peak at 11.670 min is identified as Sample 3. Therefore, Sample A contains impurities

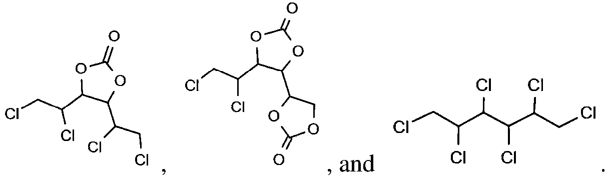

### 2. Characterization of impurities in the product (Sample B) prepared in Example 2

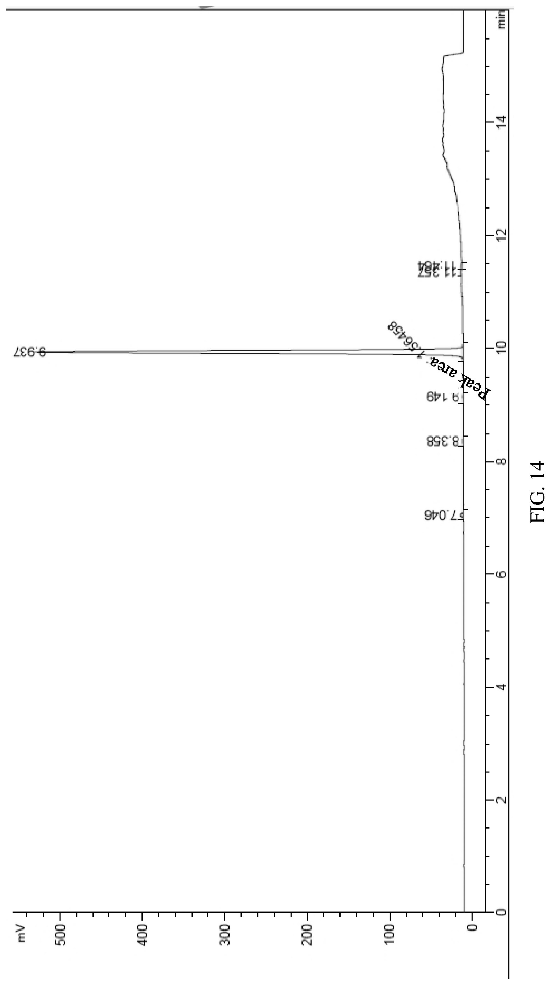

Sample B was tested by the liquid chromatography to obtain a chromatogram in FIG. 14.

A main peak in the chromatogram is the target product. A peak at 9.149 min corresponds to Sample 2. A peak at 11.484 min corresponds to Sample 1. No peak appears at a position corresponding to Sample 3 in the chromatogram.

It can be seen from the chromatogram in FIG. 14 that Sample B contains impurities 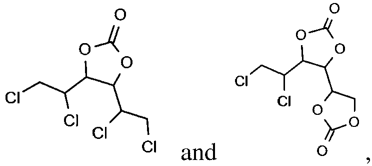 but does not contain impurity

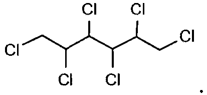

In the description of the present disclosure, the description with reference to the terms "one embodiment," "some embodiments," "an example," "a specific example," or "some examples," etc., means that specific features, structures, materials, or characteristics described in conjunction with the embodiment(s) or example(s) are included in at least one embodiment or example of the present disclosure. In the present disclosure, any illustrative reference of the above terms does not necessarily refer to the same embodiment(s) or example(s). Moreover, the specific features, structures, materials, or characteristics as described can be combined in any one or more embodiments or examples as appropriate. In addition, different embodiments or examples and features of different embodiments or examples described in the specification may be combined by those skilled in the art without mutual contradiction.

Although the embodiments of the present disclosure have been shown and described above, it should be understood that the above-mentioned embodiments are exemplary and should not be construed as limiting the present disclosure. Those skilled in the art can make changes, modifications, substitutions, and alternations to the above-mentioned embodiments within the scope of the present disclosure.

## Claims

1. A polycyclic compound, comprising:
a compound represented by Formula I; and
a chlorine-containing organic compound,
wherein based on a mass of the polycyclic compound, a content of the compound represented by the Formula I is greater than or equal to 99 wt%, and a total content of the chlorine-containing organic compound is less than or equal to 300 ppm, 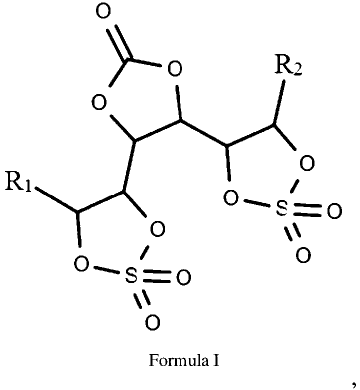
where in the Formula I, R₁ and R₂ are each independently selected from any one of H, F, alkyl, or fluoroalkyl.

2. The polycyclic compound according to claim 1, satisfying at least one of:
(a) the chlorine-containing organic compound comprises a chlorine-containing organic compound without cyclic sulfate groups and cyclic carbonate groups, a chlorine-containing organic compound containing only cyclic carbonate groups, a chlorine-containing organic compound containing only cyclic sulfate groups, or a chlorine-containing organic compound containing 1 to 2 cyclic carbonate groups and 1 cyclic sulfate group;
(b) R₁ and R₂ are each independently selected from any one of H, F, or fluoromethyl;
(c) based on the mass of the polycyclic compound, the total content of the chlorine-containing organic compound is less than or equal to 100 ppm; or
(d) based on the mass of the polycyclic compound, the content of the compound represented by the Formula I is greater than 99.9 wt%.

3. The polycyclic compound according to claim 1 or 2, satisfying at least one of:
(i) the chlorine-containing organic compound comprises one or more of 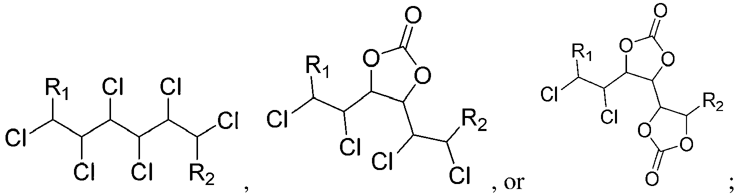
(ii) the polycyclic compound further comprises other organic compounds, the other organic compounds being selected from one or more of the following 6 compounds: 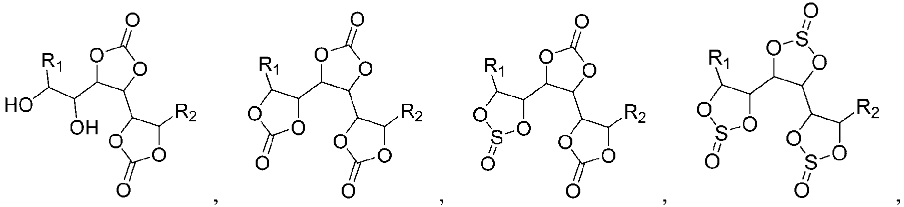 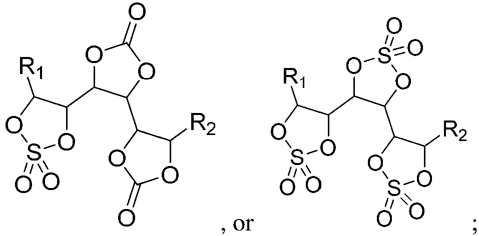 or
(iii) the compound represented by the Formula I comprises

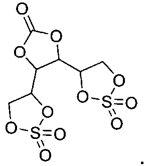

4. The polycyclic compound according to claim 3, wherein:
based on the mass of the polycyclic compound, a total content of the other organic compounds is less than or equal to 1000 ppm; and/or
based on the mass of the polycyclic compound, a total content of 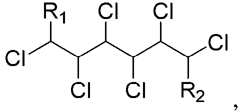 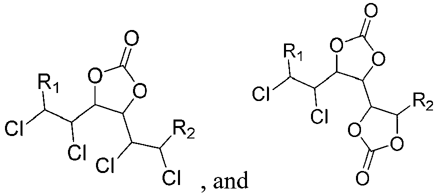 is less than or equal to 50 ppm.

5. The polycyclic compound according to claim 3 or 4, wherein based on the mass of the polycyclic compound, a total content of the other organic compounds is less than or equal to 200 ppm.

6. A method for preparing a polycyclic compound, the method comprising:
(1) mixing a hexahydric alcohol, a monohydric alcohol, a carbonate, and a basic catalyst, and performing a transesterification reaction at a temperature ranging from 65°C to 100°C under a pressure controlled to be less than 2.5 MPa for 0.5 hours to 6 hours to obtain a compound 1;
(2) dissolving the compound 1 in a solvent, and performing a condensation reaction with thionyl chloride to obtain a compound 2; and
(3) reacting the compound 2 with an oxidizing agent to obtain a compound represented by Formula I,
wherein structural formulas of the hexahydric alcohol, the compound 1, and the compound 2 are represented by: 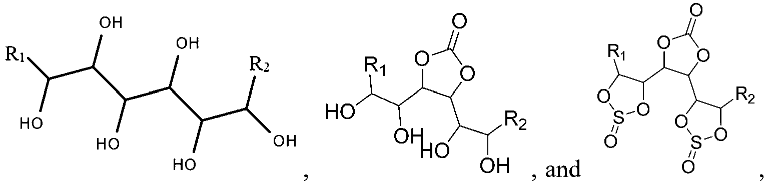 respectively, and R₁ and R₂ are each independently selected from any one of H, F, alkyl, or fluoroalkyl; and
the compound represented by the Formula I is 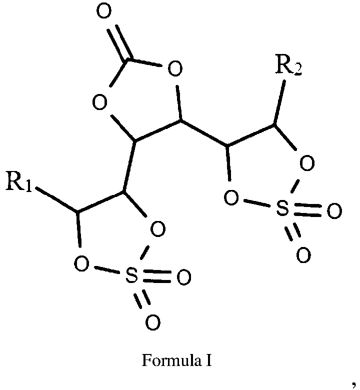
where in the Formula I, R₁ and R₂ are each independently selected from any one of H, F, alkyl, or fluoroalkyl.

7. The method according to claim 6, wherein the step (1) satisfies at least one of:
(A) subsequent to completion of the transesterification reaction, the method further comprises: performing a reduced-pressure treatment to remove a low-boiling component;
(B) the transesterification reaction is performed at a temperature ranging from 70°C to 90°C and a pressure ranging from 0.5 MPa to 2.2 MPa for 0.5 hours to 6 hours;
(C) a molar ratio of the carbonate to the hexahydric alcohol ranges from 3:1 to 5:1;
(D) a molar ratio of the monohydric alcohol to the hexahydric alcohol ranges from 10:1 to 20:1;
(E) based on a mass of the hexahydric alcohol, an amount of the basic catalyst ranges from 0.01 wt% to 5 wt%;
(F) the hexahydric alcohol comprises one or more of mannitol, sorbitol, or galactitol;
(G) the carbonate comprises one or more of dimethyl carbonate, diethyl carbonate, ethyl methyl carbonate, or diphenyl carbonate;
(H) the monohydric alcohol comprises one or more of methanol, ethanol, propanol, or butanol; or
(I) the basic catalyst comprises one or more of sodium hydroxide, potassium hydroxide, potassium carbonate, sodium carbonate, lithium carbonate, sodium methoxide, sodium ethoxide, sodium tert-butoxide, potassium tert-butoxide, triethylamine, or pyridine.

8. The method according to claim 6 or 7, satisfying at least one of:
(I) in the step (2), subsequent to completion of the condensation reaction, the method further comprises: performing a reflux deacidification treatment;
(II) in the step (2), subsequent to dissolving the compound 1 in the solvent, thionyl chloride is added dropwise to perform the condensation reaction;
(III) in the step (2), the condensation reaction is performed at a temperature ranging from 20°C to 100°C for 1 hour to 4 hours;
(IV) in the step (2), a molar ratio of the compound 1 to the thionyl chloride is 1:(2 to 3), and an addition duration of the thionyl chloride ranges from 0.5 hours to 2 hours;
(V) in the step (2), the solvent comprises one or more of an ether solvent, N, N-dimethylformamide, N,N-dimethylacetamide, or an ester solvent; or
(VI) in the step (3), the oxidizing agent is provided as an aqueous solution, and subsequent to reacting the compound 2 with the oxidizing agent, the method further comprises: performing a heating treatment on a reaction product to remove water.

9. The method according to any one of claims 6 to 8, wherein in the step (2), subsequent to completion of the condensation reaction, the method further comprises a reflux deacidification treatment, where:
a temperature of the reflux deacidification treatment rangs from 40°C to 80°C; and/or
a duartion of the reflux deacidification treatment ranges from 10 minutes to 50 minutes.

10. The method according to any one of claims 6 to 9, wherein the step (1) satisfies at least one of:
1) the transesterification reaction is performed at a temperature ranging from 75°C to 85°C and a pressure ranging from 1 MPa to 2 MPa;
2) a molar ratio of the monohydric alcohol to the hexahydric alcohol ranges from 12:1 to 18:1; or
3) based on a mass of the hexahydric alcohol, an amount of the basic catalyst ranges from 0.02 wt% to 0.5 wt%.

11. The method according to any one of claims 6 to 10, wherein the step (1) satisfies at least one of:
(α) a molar ratio of the monohydric alcohol to the hexahydric alcohol ranges from 14:1 to 16:1; or
(β) based on a mass of the hexahydric alcohol, an amount of the basic catalyst ranges from 0.02 wt% to 0.1 wt%.

12. A polycyclic compound prepared by the method according to any one of claims 5 to 11, the polycyclic compound comprising:
the compound represented by the Formula I; and
a chlorine-containing organic compound,
wherein a content of the compound represented by the Formula I is greater than or equal to 99 wt%, and a total content of the chlorine-containing organic compound is less than or equal to 300 ppm.

13. Use of the polycyclic compound according to any one of claims 1 to 4 or the polycyclic compound prepared by the method according to any one of claims 5 to 12 in the field of electrolyte and battery.

14. An electrolyte, comprising an electrolyte additive, wherein the electrolyte additive comprises the polycyclic compound according to any one of claims 1 to 4 or the polycyclic compound prepared by the method according to any one of claims 5 to 12.

15. The electrolyte according to claim 14, wherein based on a total mass of the electrolyte, a content of the electrolyte additive ranges from 0.1 wt% to 5 wt%.

16. A battery, comprising the electrolyte according to claim 14 or 15.
